(19) Europäisches Patentamt
European Patent Office
Office européen des brevets

(11) Numéro de publication: 0 016 673
B1

(12) FASCICULE DE BREVET EUROPEEN

(45) Date de publication du fascicule du brevet: 10.02.82

(51) Int. Cl.³: C 07 C 93/04

(21) Numéro de dépôt: 80400250.9

(22) Date de dépôt: 22.02.80

# (54) Procédé de solubilisation de sels organiques ou minéraux dans des solvants organiques et complexes de sels organiques ou minéraux.

(30) Priorité: 02.03.79 FR 7905438

(43) Date de publication de la demande:
01.10.80 Bulletin 80/20

(45) Mention de la délivrance du brevet:
10.02.82 Bulletin 82/6

(84) Etats contractants désignés:
AT BE CH DE FR GB IT LU NL SE

(56) Documents cités:
FR - A - 1 302 365
(WYANDOTTE CHEMICAL CORP.)
*Page 1, lignes 1—12; résumé; exemples*

CHEMICAL ABSTRACTS, vol. 59, no 6, 16 septembre 1963 colonne 5850d, e
Columbus, Ohio, US
V. G. TIMOSHEV et al. "The importance of the structure and physical properties of molecules of extractants".

(73) Titulaire: RHONE-POULENC INDUSTRIES
Brevets Pharma 25 Quai Paul Doumer
F-92408 Courbevoie Cedex (FR)

(72) Inventeur: Soula, Gérard
33, rue Nungesser
F-69330 Meyzieu (FR)

(74) Mandataire: Cazes, Jean-Marie et al.
RHONE POULENC Service Brevets Chimie et Polymères B.P. 753
F-75360 Paris Cedex 08 (FR)

(56) Documents cités :
CHEMICAL ABSTRACTS, vol. 80, 1974. abstract 122681v
Columbus, Ohio, USA
MATSUDA, M et al. "Synthesis and the applications of polyalkylene glycol derivatives. XII. Synthesis and surface active properties of tertiary ether amines".

J. ORG. CHEM., vol. 40, no. 9, 1975.
Washington D.C. (US)
C. G. KRESPAN: "The oxetane function spiro to polyoxaaza macroheterocycles". pages 1205—1209.

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 016 673 B1

Courier Press, Leamington Spa, England.

# Procédé de solubilisation de sels organiques ou minéraux dans des solvants organiques et complexes de sels organiques ou minéraux

La présente invention a pour objet un procédé pour solubiliser un sel organique ou minéral; elle concerne plus particulièrement un procédé pour solubiliser un sel organique ou minéral dans un solvant organique dans lequel le sel organique ou minéral n'est pas soluble ou pour augmenter la solubilité d'un sel organique ou minéral dans un solvant organique.

Ce problème de solubilisation est important car on sait bien que les sels concernés par la présente invention soit, sont insolubles soit, n'ont pas une solubilité toujours satisfaisante dans la plupart des solvants organiques utilisés industriellement.

Les solvants organiques peuvent être classés selon leur aptitude à dissoudre un sel.

Cette classification tient compte de divers facteurs comme, notamment, le caractère polaire et le caractère protique de ces solvants. L'homme de l'art sait bien que les solvants polaires, c'est-à-dire les solvants ayant une constante diélectrique élevée, dissolvent plus facilement les sels que les solvants à caractère apolaire, c'est-à-dire ayant une constante diélectrique faible. Il est également bien connu que les meilleurs solvants polaires sont les solvants polaires protiques, c'est-à-dire les solvants ayant une constante diélectrique élevée et possédant des atomes d'hydrogène à caractère acide.

C'est ainsi que les meilleurs solvants organiques pour dissoudre les sels sont, notamment, la formamide, l'acétamide, l'acide formique, l'hexaméthylphosphorotriamide (H.M.P.T.), le diméthylsulfoxyde, le sulfolane, le N-méthylpyrrolidone, la diméthylformamide, la diméthylacétamide, le méthanol et l'acétone, les moins bons étant, notamment, le chloroforme, le chlorure de méthylène, le tétrachlorure de carbone, le trichloroéthylène, le dichloroéthane, le chlorobenzène, l'orthodichlorobenzène, le benzène, le toluène, les xylènes, le cyclohexane et l'hexane.

Il faut noter que la difficulté de mise en oeuvre industrielle de ces solvants croît généralement lorsque l'on va des moins bons solvants aux meilleurs solvants. En effet, les meilleurs solvants peuvent réagir avec les sels à dissoudre et donner des produits secondaires. Certains sont très toxiques comme l'HMPT, d'autres comme le diméthylsulfoxyde sont malodorants et peu stables thermiquement. De plus, tous ces produits sont chers. Il est clair que l'homme de l'art préfère, quand cela est possible, utiliser un solvant apolaire comme le toluène et les xylènes par exemple qui sont peu onéreux et d'un maniement plus aisé compte tenu de leur faible toxicité et de leur stabilité thermique et chimique.

Pourtant, un sel donné pourra être, et ce sera le cas le plus fréquent, pratiquement totalement insoluble dans tous les solvants apolaires. Il ne le sera que partiellement dans les solvants protiques polaires, son maximum de solubilité étant atteint avec les meilleurs des solvants protiques polaires.

On voit donc l'intérêt qu'il y aurait à solubiliser un sel dans un solvant dans lequel il est initialement insoluble quand ce solvant est plus facile à mettre en oeuvre industriellement ou à augmenter sa solubilité dans le même solvant.

C'est ce double objectif que permet d'atteindre la présente invention, objectif dont on verra plus loin les importantes applications au plan industriel.

On connait dans l'art antérieur un certain nombre de travaux ayant conduit à des solutions partielles dans ce domaine.

C'est ainsi que la demande de brevet français 69.43879 publié sous le numéro 2 026 481 décrit des composés polyéthers macrocycliques. Ces composés peuvent former des complexes avec les cations de certains composés métalliques, en particulier avec les sels de métaux alcalins et de métaux alcalino-terreux. Ces complexes sont des réactifs analytiques pour l'utilisation dans des milieux non hydroxylés dans lesquels les composés non complexés de métaux sont normalement insolubles. Les composés macrocycliques décrits dans cette demande de brevet français ont de 15 à 30 atomes dans le cycle polyéther et sont constitués de 5 à 10 unités —O—X— dans lesquelles X, pour un composé particulier, est soit (a) $-CHR_1-CHR_2-$ soit (b) $-CHR_1-CR_3R_4-CHR_2-$ où $R_1$ $R_2$ $R_3$ $R_4$ sont un atome d'hydrogène ou un radical alkyle.

Comme on le voit, ces composés plus communément appelés "éthers couronnes" ont une structure très sophistiquée. Il en découle que le procédé de préparation est d'une mise en oeuvre délicate. Un des inconvénients majeurs de l'utilisation de ces éthers-couronnes est sans aucun doute leur prix de revient qui est extrêmement élevé. Un autre inconvénient important est que sur le plan pratique, d'après la demande de brevet précitée, ils ne sont utilisables qu'avec les composés des métaux alcalins et avec les composés des métaux alcalino-terreux ayant un poids atomique supérieur à 40.

Une autre demande de brevet français, la demande 70.21079 publiée sous le numéro 2 052 947 décrit également des composés macrobicycliques susceptibles de complexer des sels en les rendant de ce fait solubles dans des solvants dans lesquels ils sont normalement insolubles. Ces composés macrobicycliques communément appelés "cryptants" ont aussi une structure extrêmement sophistiquée qui implique les inconvénients soulignés dans le cas des éthers couronnes.

On connaît encore la référence Chemical Abstracts 595850 d.e. Vol. 59, qui décrit l'extraction de certains métaux (ion uranyle, $Pu^{4+}$, $Zr^{4+}$, $Nb^{4+}$) en solution nitrique. L'extraction se fait à l'aide d'un composé qui peut être $(RO-CH_2-CH_2)_3N$, où R est un radical alkyle ayant de 1 à 4 atomes de carbone ou le radical phényle. L'extraction se fait, par exemple dans le cas de l'ion uranyle, par l'intermédiaire de

$(RO—CH_2—CH_2)_3\ N^+H\ [UO_2(NO_3)_3]^-$. Du fait du milieu acide nitrique, l'agent d'extraction est l'ion ammonium $(RO—CH_2—CH_2)_3\ N^+H$ et l'ion uranyle est sous forme anionique $UO_2(NO_3)_3^-$. Le principal inconvénient de ce type de procédé est, d'une part, la nécessité d'un milieu acide pour que l'extraction puisse avoir lieu et, d'autre part, le fait qu'il ne permet pas l'extraction de métaux non susceptibles de se mettre sous forme anionique.

Ce qui précède met nettement en évidence le besoin réel qui subsiste au plan industriel: pouvoir disposer d'un procédé de mise en oeuvre simple, pour solubiliser un sel minéral ou organique dans un solvant organique dans lequel ce sel n'est initialement pas soluble ou pour augmenter la solubilité d'un sel minéral ou organique dans un solvant organique.

Les travaux de la demanderesse ont permis de mettre ou point ce procédé.

La présente invention a donc pour objet un procédé pour solubiliser un sel organique ou minéral dans un solvant organique dans lequel le sel n'est initialement pas soluble ou pour augmenter la solubilité d'un sel organique ou minéral dans un solvant organique, caractérisé en ce que l'on met en présence,. le sel organique ou minéral de formule $A^- M^+$ dans laquelle $A^-$ est un anion organique ou minéral et $M^+$ est un cation choisi parmi le groupe comprenant le cation $NH_4^+$ et ses dérivés et les cations dérivés des métaux des groupes $I_A$, $II_A$, $III_A$, $IV_A$, $V_A$, $VI_A$, $VII_A$, VIII, $I_B$, $II_B$, $III_B$, $IV_B$, et $V_B$ de la classification periodique des éléments et au moins un agent séquestrant soluble dans le solvant organique de formule:

$$N[—CHR_1—CHR_2—O—(CHR_3—CHR_4—O)_n—R_5]_3 \qquad (I)$$

dans laquelle n est un nombre entier supérieur ou égal à 0 et inférieur ou égal à 10 environ ($0 \leqslant n \leqslant 10$),

$R_1$ $R_2$ $R_3$ $R_4$ identiques ou différents représentent un atome d'hydrogène ou un radical alkyle ayant de 1 à 4 atomes de carbone,

$R_5$ représente un radical alkyle ou cycloalkyle ayant de 1 à 12 atomes de carbone, un radical phényle ou un radical de formule $—C_mH_{2m}—\emptyset$ ou $C_mH_{2m+1}—\emptyset—$, m étant compris entre 1 et environ 12,

l'agent séquestrant formant avec le sel un complexe de formule:

$$[N\text{-}[CHR_1—CHR_2—O—(CHR_3—CHR_4—O)_n—R_5]_3]_y\ (M^+ A^-) \qquad (II)$$

dans laquelle y est supérieur ou égal à 1 et inférieur ou égal à 3 ($1 \leqslant y \leqslant 3$), ce complexe étant soluble dans le solvant organique.

Selon une première variante, on met en présence en une seule étape, le sel organique ou minéral anhydre et l'agent séquestrant de formule (I) en solution dans ledit solvant organique.

Selon une deuxième variante, on met en présence en une seule étape, le sel organique ou minéral en solution aqueuse et l'agent séquestrant de formule (I) en solution dans ledit solvant organique, ledit solvant organique étant dans cette variante non miscible à l'eau.

Selon une troisième variante dans une première étape, on met en présence le sel minéral ou organique anhydre en solution dans un tiers solvant et l'agent séquestrant de formule (I) en solution dans le même tiers solvant, dans une deuxième étape on élimine le tiers solvant et dans une troisième étape, on met en présence le produit résultant de la deuxième étape et ledit solvant organique.

Selon une quatrième variante, on met en présence dans une première étape le sel minéral ou organique anhydre et l'agent séquestrant de formule (I) en l'absence de tout solvant et dans une deuxième étape, on met en présence le produit résultant de la première étape et ledit solvant organique.

Ces quatre variantes correspondent à des mises en oeuvre différentes du procédé selon l'invention. L'homme de l'art choisira parmi ces quatre variantes suivant la nature du problème qu'il aura à résoudre compte tenu, notamment, de la nature du sel à solubiliser.

La troisième variante permet l'isolement aisé du complexe de formule II obtenu à la fin de la deuxième étape.

La quatrième variante permet également l'isolement du complexe de formule II à la fin de la première étape, sans qu'il soit nécessaire d'avoir recours à un tiers solvant.

Les agents séquestrants préférés pour la mise en oeuvre du procédé selon l'invention sont ceux pour lesquels $R_1$ $R_2$ $R_3$ et $R_4$ représentent un atome de hydrogène ou un radical méthyle, $R_5$ et n ayant la signification précédente.

Parmi ces derniers, on préfère encore plus particulièrement mettre en oeuvre les agents séquestrants pour lesquels n est supérieur ou égal à 0 et inférieur ou égal à 6 et pour lesquels $R_5$ représente un radical alkyle ayant de 1 à 4 atomes de carbone.

On peut citer:

—la tris(oxa-3 butyl)amine de formule:

$N—(CH_2—CH_2—O—CH_3)_3$

—la tris(dioxa-3,6 heptyl)amine de formule:
$N—(CH_2—CH_2—O—CH_2—CH_2—O—CH_3)_3$
—la tris(trioxa-3,6,9 décyl)amine de formule:
$N—(CH_2—CH_2—O—CH_2—CH_2—O—CH_2—CH_2—O—CH_3)_3$
—la tris(dioxa-3,6 octyl)amine de formule:
$N—(CH_2—CH_2—O—CH_2—CH_2—O—C_2H_5)_3$
—la tris(trioxa-3,6,9 undécyl)amine de formule:
$N—(CH_2—CH_2—O—CH_2—CH_2—O—CH_2—CH_2—O—C_2H_5)_3$
—la tris(dioxa-3,6 nonyl)amine de formule:
$N—(CH_2—CH_2—O—CH_2—CH_2—O—C_3H_7)_3$
—la tris(trioxa-3,6,9 dodécyl)amine de formule:
$N—(CH_2CH_2—O—CH_2—CH_2—O—CH_2—CH_2—O—C_3H_7)_3$
—la tris(dioxa-3,6 décyl)amine de formule:
$N—(CH_2—CH_2—O—CH_2—CH_2—O—C_4H_9)_3$
—la tris(trioxa-3,6,9 tridécyl)amine de formule:
$N—(CH_2—CH_2—O—CH_2—CH_2—O—CH_2—CH_2—O—C_4H_9)_3$
—la tris(tétraoxa-3,6,9,12 tridecyl)amine de formule:
$N—(CH_2—CH_2—O—CH_2—CH_2—O—CH_2—CH_2—O—CH_2—CH_2—O—CH_3)_3$
—la tris(hexaoxa-3,6,9,12,15,18 nonadécyl)amine de formule:
$N—[(CH_2—CH_2—O)_6—CH_3]_3$

On peut encore citer:

—la tris(dioxa-3,6 méthyl-4 heptyl)amine de formule:
$N—(CH_2—CH_2—O—CH(CH_3)—CH_2—O—CH_3)_3$
—la tris(dioxa-3,6 diméthyl-2,4 heptyl)amine de formule:
$N—(CH_2—CH(CH_3)—O—CH(CH_3)—CH_2—O—CH_3)_3$

Dans la définition de l'invention qui est donnée ci-dessus, il est clair que $M^+$ représente aussi bien les cations monovalents que les cations polyvalents. Il en est de même en ce qui concerne l'anion $A^-$.

Alors que les travaux de l'art antérieur conduisait le chercheur à orienter ses travaux vers des molécules complexantes de structure cyclique très compliquée, la demanderesse a découvert que des molécules beaucoup plus simples non cycliques d'accès plus facile permettaient d'obtenir d'excellents résultats.

Les amines utilisées dans le procédé selon l'invention sont connues en tant que telles dans l'art antérieur. C'est ainsi que le brevet français 1 302 365 cite l'obtention des amines tertiaires $N—(CH_2—CH_2—O—CH_3)$ et $N—(CH_2—CH_2—O—CH_2—CH_2—O—CH_3)_3$ comme sous produit de la synthèse des amines primaires et secondaires correspondantes, ces amines primaires et secondaires étant des produits intéressants comme intermédiaires en vue de la synthèse de substances pharmaceutiques, comme inhibiteurs de corrosion, comme intermédiaires en vue de la synthèse de produits chimiques intéressants en agriculture et comme émulsifiants. Il n'est pas inutile de souligner que le domaine d'application des composés obtenus dans le brevet 1 302 365 précité simultanément aux amines utilisées dans le procédé objet de la présente demande est totalement étranger au domaine de l'invention.

Le procédé de l'invention est particulièrement adapté à la solubilisation des sels $A^-$ $M^+$ dans lesquels $A^-$ est un anion organique ou minéral et $M^+$ est un cation choisi parmi le groupe comprenant:

—$NH_4^+$ et ses dérivés $RNH_3^+$ où R est un radical alkyle ou aryle
—et les cations dérivés des métaux
. Li, Na, K, Rb, Cs
. Mg, Ca, Sr, Ba
. Sc, Y, La et lanthanides, Ac et actinides
. Ti. Zr, Hf,
. V, Nb, Ta
. Cr, Mo, W
. Mn, Tc, Re
. Fe, Co, Ni
. Ru, Rh, Pd
. Os, Ir, Pt
. Cu, Ag, Au
. Zn, Cd, Hg,
. Al, Ga, In, Tl
. Ge, Sn, Pb
. Sb, Bi

Cette préférence ne limite aucunement l'invention; elle dérive uniquement de l'intérêt du procédé selon l'invention au plan industriel.

Ce même intérêt conduit à considérer le procéde selon l'invention plus particulièrement, mais non exclusivement, adapté à la solubilisation de sels $A^-$ $M^+$ dans lesquels $A^-$ est choisi parmi le groupe comprenant:

—d'une part les anions minéraux: $SNC^-$, $O{=}C{=}N^-$, $Cl^-$,$Br^-$, $H^-$, $NO_3^-$, $I^-$, $F^-$, $CN^-$, $SH^-$, $S^-$, $OH^-$, $HSO_3^-$, $ClO_4^-$, $BrO_4^-$, $NH_2^-$, $NO_2^-$, $BF_4^-$, $BrO^-$, $ClO^-$, $BH_4^-$, $SO_3^{--}$, $PO_4^3$, $CO_3^{--}$, $SO_4^{--}$, $ClO_3^-$, $BrO_3^-$, $AlH_4^-$,

—et d'autre part les anions organiques dérivés

. des alcools aliphatiques comme par exemple le méthanol ($CH_3O^-$) et ses homologues supérieurs, le cyclopentanol ($C_5H_9O^-$) le cyclohexanol ($C_6H_{11}O^-$), l'alcool benzylique (Ø—$CH_2$—$O^-$) et ses homologues supérieurs.

. des phénols comme par exemple, le phénol ($ØO^-$) et ses dérivés comme l'acide picrique

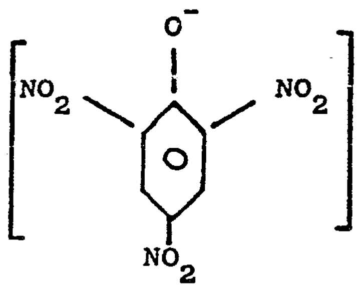

les naphtols comme l'$\alpha$ naphtol

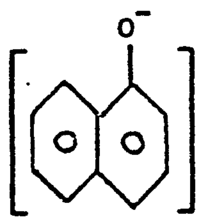

et le $\beta$-naphtol

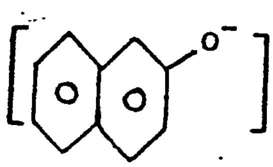,

les phénols substitués par exemple

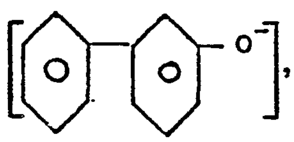,

les diphénols par exemple

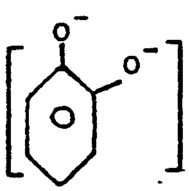

les bisphénols

par exemple 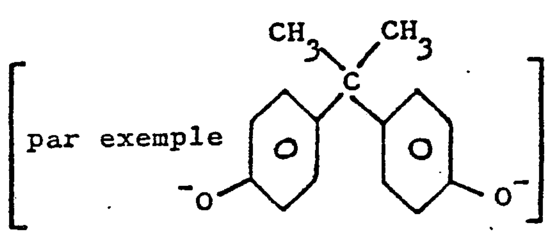

. des thiols comme par exemple, le méthylmercaptan ($CH_3S^-$) et ses homologues supérieurs, le benzylmercaptan (Ø—$CH_2$—$S^-$) et ses homologues,

. des thiophénols comme par exemple le thiophénol ($ØS^-$) les alkylthiophénols (par exemple

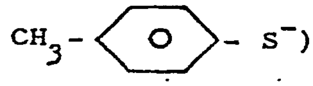

. des acides comme par exemple l'acide acétique ($CH_3COO^-$), ses homologues supérieurs et ses dérivés comme par exemple l'acide cyanoacétique ($CNCH_2COO^-$) et l'acide chloroacétique ($ClCH_2COO^-$), les acides versatiques (par exemple

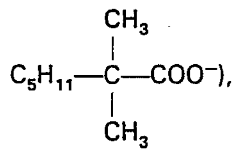

les acides phénylacétiques (Ø—$CH_2$—$COO^-$ et ses homologues), les acides phénoxypropioniques (Ø—$CH_2$—$COO^-$ et ses homologues), les acides phénoxypropioniques

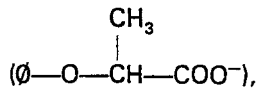

les acides benzoïques (Ø—$CO_2^-$, Ø$(CO_2^-)_2$ par exemple), les acides naphténiques

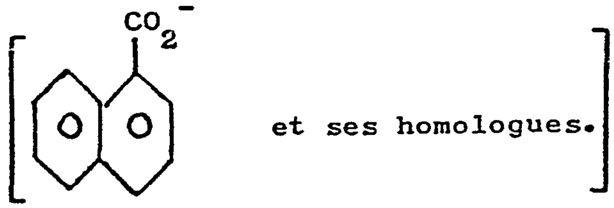


. des acides sulfoniques comme par exemple l'acide méthane sulfonique ($CH_3$—$SO_3^-$) et ses homologues supérieurs, l'acide benzène sulfonique (Ø—$SO_3^-$) et ses homologues, l'acide naphtyl sulfonique

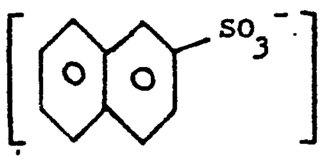

et ses homologues.

. des amines comme par exemple les amines aliphatiques ($CH_3NH^-$ et ses homologues supérieurs), les anilines (par exemple Ø—$NH^-$ et ses homologues,

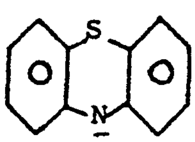 , 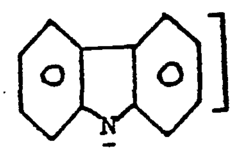

les benzylamines (Ø—$CH_2$—$NH^-$ et ses homologues)

. des amides comme par exemple les amides aliphatiques ($CH_3CONH^-$ et ses homologues supérieurs), les amides aromatiques (Ø—$CONH^-$ et ses homologues),

. des composés organiques à hydrogène mobile comme par exemple les esters maloniques [$\bar{C}H$—$(CO_2CH_3)_2$ par exemple], le chloroacétonitrile (Cl—$\bar{C}$HCN) et ses homologues, les phénylacétonitriles (Ø—$\bar{C}$H—CN par exemple), le triphényl méthane ($Ø_3\bar{C}$), le chloroforme ($\bar{C}$ $Cl_3$), les acétylacétonates d'alkyle

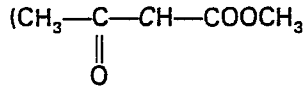 par exemple),

la phénylacétone

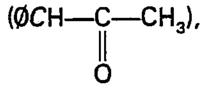

les acétophénones

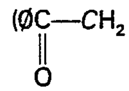 par exemple),

. des silanols comme par exemple

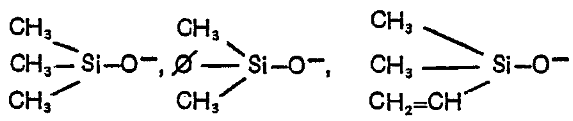

Il est important de souligner que ces exemples d'anions ne sont pas limitatifs. Tout sel dont le cation répond à la définition qui a été donnée précédemment peut être traité selon le procédé de l'invention.

Le choix de l'agent séquestrant le plus adapté à la solubilisation d'un sel donné doit être fait en tenant compte principalement du cation $M^+$: plus la taille du cation sera importante, plus le nombre d'atomes d'oxygène contenus dans la molécule de l'agent séquestrant devra être élevé. Par exemple, le picrate de potassium en solution aqueuse mélangé intimement avec du chlorure de méthylène ne se solubilise pas dans ce solvant. Si on ajoute un agent séquestrant selon le procédé de l'invention, on observe une solubilisation. Cette solubilisation sera plus importante avec la tris(trioxa-3,6,9 décyl)amine-contenant 3 atomes d'oxygène par chaine branchée sur l'atome d'azote- qu'avec la tris(dioxa-3,6 heptyl)amine qui ne contient que 2 atomes d'oxygène par chaine branchée sur l'atome d'azote. Par contre, pour le picrate de sodium (le cation $Na^+$ est plus petit que le cation $K^+$) la meilleure solubilisation sera obtenue avec la tris(dioxa-3,6 heptyl)amine.

Le solvant doit répondre à un certain nombre de conditions: il faut d'abord qu'il solubilise l'agent séquestrant; il faut aussi qu'il soit inerte chimiquement vis-à-vis du sel à dissoudre (de la même façon, il faut que l'agent séquestrant n'agisse qui comme complexant vis-à-vis du sel minéral ou organique).

Il faut aussi souligner que pour obtenir le meilleure solubilisation, plus le solvant aura un caractère apolaire marqué, plus l'agent séquestrant devra avoir un caractère lipophyle marqué (c'est-à-dire plus l'agent séquestrant devra contenir d'atomes de carbone).

Toujours pour obtenir la meilleure solubilisation, plus l'anion $A^-$ aura une forte densité électronique, plus le solvant devra être polaire. Les anions à forte densité électronique — anions "durs" — sont des anions de petite taille comme par exemple $OH^-$, $F^-$, $Cl^-$. Les anions "mous" de plus grosse taille sont par exemple $SCN^-$ Ø—$O^-$,

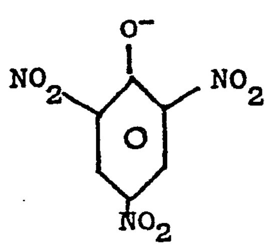

Les agents séquestrants utilisés dans le procédé selon l'invention sont solubles dans tous les solvants organiques usuels.

Le procédé selon l'invention permet la solubilisation des sels visés notamment dans les solvants suivants pris isolement ou en mélange: les solvants aliphatiques tels que l'hexane, le cyclohexane, les solvants aromatiques comme le benzène, le toluène l'o-xylène, le m-xylène, le p-xylène, les solvants aromatiques halogénés comme le chlorobenzène, l'o-dichlorobenzène, le trichloro-1,2,4 benzène, les solvants aliphatiques halogénés comme le chloroforme, le chlorure de méthylene, le tétrachlorure de carbone, le dichloro-1,2 éthane, le trichloro-1,1,1 éthane, le trichloro-1,1,2 éthane, le trichloro-1,2,2

trifluoro-1,1,2 éthane, les solvants oléfiniques halogénés comme le perchloroéthylène. L'invention permet également la mise en solution dans l'acétone, l'acétonitrile, la diméthylformamide, la N-méthylpyrrolidone, la diméthylacétamide, l'hexaméthylphosphorotriamide, le diméthylsulfoxyde, le sulfolane, le méthanol, l'éthanol et l'isopropanol.

La température à laquelle est mise en oeuvre le procédé peut être importante. En effet, un complexe sel-agent séquestrant pourra être insoluble à froid dans le solvant retenu mais soluble à chaud. D'autre part, la température sera limitée par la température d'ébullition du solvant utilisé. D'une façon générale, le température peut varier dans de larges limites; elle est plus particulièrement comprise entre −50 et 250°C environ.

La pression à laquelle on opère n'est pas critique. On peut opérer sous pression atmosphérique ou sous pression inférieure ou supérieure à la pression atmosphérique.

On utilise généralement l'agent séquestrant en quantité telle que le rapport molaire de l'agent séquestrant au sel à solubiliser est compris entre environ 0,001 et environ 50. Généralement, plus la quantité d'agent séquestrant ser élevée, plus la complexation aura lieu et plus la dissolution sera grande. Au-dessus du rapport 50, l'augmentation de solubilité n'est plus significative.

Le procédé selon l'invention peut être appliqué dans un grand nombre de domaines de l'industrie chimique.

Il est clair pour l'homme de l'art que la solubilisation obtenue selon le procédé de l'invention permet la mise en réaction du sel considéré avec un substrat dans des solvants dans lesquels cette réaction ne pouvait être effectuée, ce qui peut être d'un grand intérêt dans de nombreux cas de synthèse organique. C'est le cas de la première variante évoquée ci-avant.

Une autre application intéressante concerne l'extraction des métaux. On peut extraire d'une solution un composé métallique $A^-M^+$ en le faisant passer par complexation selon l'invention d'une phase organique ou aqueuse dans une autre phase organique. C'est le cas de la deuxième variante. Par exemple, il est ainsi possible d'extraire d'une solution aqueuse des picrates de métaux alcalins et alcalino-terreux ainsi que le picrate d'Ag par un agent séquestrant selon l'invention dans le chlorure de méthylène. On peut également extraire de la même façon les thiocyanates alcalins.

La présente invention concerne aussi en tant que produits nouveaux les complexes de formule:

$$[N-[CHR_1-CHR_2-O-(CHR_3-CHR_4-O-)_nR_5]_3]_y(M^+\ A^-) \qquad (II)$$

où $R_1$ $R_2$ $R_3$ $R_4$ $R_5$, n, $M^+$ $A^-$, et $y_1$ se définissent d'une façon générale et d'une façon préférentielle comme précédemment.

Encore plus particulièrement, l'invention concerne les complexes suivants:

$[N-(CH_2-CH_2-O-CH_3)_3]_y(A^-M^+)$
$[N-(CH_2-CH_2-O-CH_2-CH_2-O-CH_3)_3]_y(A^-M^+)$
$[N-(CH_2-CH_2-O-CH_2-CH_2-O-CH_2-CH_2-O-CH_3)_3)_y(A^-M^+)$
$[N-(CH_2-CH_2-O-CH_2-CH_2-O-C_2H_5)_3]_y(A^-M^+)$
$[N-(CH_2-CH_2-O-CH_2-CH_2-O-CH_2-CH_2-O-C_2H_5)_3]_y(A^-M^+)$
$[N-(CH_2-CH_2-O-CH_2-CH_2-O-C_3H_7)_3]_y(A^-M^+)$
$[N-(CH_2-CH_2-O-CH_2-CH_2-O-CH_2-CH_2-O-C_3H_7)_3]_y(A^-M^+)$
$[N-(CH_2-CH_2-O-CH_2-CH_2-O-C_4H_9)_3]_y(A^-M^+)$
$[N-(CH_2-CH_2-O-CH_2-CH_2-O-CH_2-CH_2-O-C_4H_9)_3]_y(A^-M^+)$
$[N-(CH_2-CH_2-O-CH_2-CH_2-O-CH_2-CH_2-O-CH_2-CH_2-O-CH_3)_3]_y(A^-M^+)$
$[N-(CH_2-CH_2-O)_6-CH_3]_3]_y(A^-M^+)$
$[N-(CH_2-CH_2-O-CHCH_3-CH_2-O-CH_3)_3]_y(A^-M^+)$
$[N-(CH_2-CHCH_3-O-CHCH_3-CH_2-O-CH_3)_3]_y(A^-M^+)$

dans lesquelles $A^-$, $M^+$ et y ont la signification précédente.

Les agents séquestrants utilisés dans le procédé selon l'invention peuvent être préparés de la façon suivante:

Ces composés peuvent être préparés par condensation d'un sel de formule:

$$R_5[O-CH(R_4)-CH(R_3)]_n-O-M$$

où $R_3$ $R_4$ $R_5$ et n ont la signification précédente et où M représente un atome de métal alcalin choisi parmi le sodium, le potassium et le lithium, soit sur une amine de formule générale:

$$N-[CH(R_1)-CH(R_2)-X]_3$$

dans laquelle $R_1$ et $R_2$ ont la signification précédente et X représente le chlore ou le brome, soit sur le chlorhydrate ou le bromhydrate correspondant.

Le rapport molaire sel de métal alcalin/amine est compris entre environ 3 et environ 5.

L'opération de condensation est réalisée à une température comprise entre 100 et 150°C pendant 1 à 15 h en présence d'un solvant qui peut être par exemple le chlorobenzène ou de préférence le monoalkyléther d'éthylène glycol de formule

$R_5—(O—CHR_4—CHR_3)_n—OH.$

On opère de préférence de telle sorte qu'on ait une solution contenant de 2 à 5 moles de sel de métal alcalin par litre de solvant.

Le mélange en fin de réaction contient principalement l'amine tertiaire de formule:

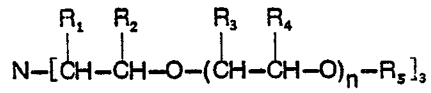

mais contient aussi en faible proportion de l'amine secondaire correspondant:

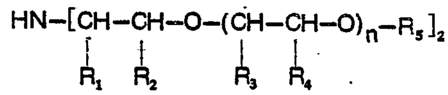

et des traces d'amine primaire:

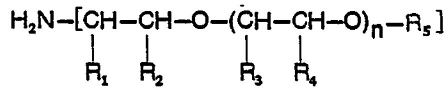

Les amines tertiaires, secondaires et primaires sont généralement respectivement dans le rapport 90:8:2 après distillation.

On peut utiliser dans le procédé selon l'invention directement le mélange ci-dessus obtenu après première distillation, c'est-à-dire contenant les trois types d'amines.

On préfère pour une meilleure mise en oeuvre de l'invention effectuer une distillation plus poussée du mélange ci-dessus afin d'obtenir une amine tertiaire sensiblement pure.

D'autres caractéristiques et avantages apparaitront à la lecture des exemples qui vont suivre. Il va de soi que ces exemples qui sont donnés uniquement à titre illustratif ne sauraient être considérés comme limitant la portée de la présente invention.

Dans ces exemples, les solubilités mesurées et les solubilités maximum calculées sont exprimées par la teneur en métal de la solution. La solubilité maximum calculée correspond au cas où tout le sel engagé passerait en solution. Le taux de solubilisation exprime le rapport de la solubilité mesurée à la solubilité maximum calculée c'est-à-dire exprime le taux de sel complexé passé en solution. Les formules des complexes données sont celles des complexes passant en solution dans le solvant considéré.

Exemple 1

Solubilisation directe de l'hexachlorure de tungstène dans le chlorure de méthylène

Dans un erlenmeyer de 50 ml équipé d'un réfrigérant ascendant et d'un agitateur magnétique, on introduit 20 ml de chlorure de méthylène anhydre et purifié (c'est-à-dire exempt de stabilisant). On ajoute ensuite 0,4 g d'hexachlorure de tungstène (0,001 mole) et 0,32 g de tris(dioxa-3,6 heptyl)amine (0,001 mole).

Après avoir agité le mélange pendant 10 mn à la température ambiante, on centrifuge et la solution claire ainsi obtenue est analysée par spectrométrie de flamme.

Solubilité mesurée: 9290 mg/l
Solubilité maximum calculée: 9295 mg/l
Taux de solubilisation: 99%
Complexe formé: $[N(CH_2—CH_2—O—CH_2—CH_2—O—CH_3)_3]$ $(WCl_6)$
*Essai comparatif:* on opère comme ci-dessus mais sans ajouter de tris(dioxa-3,6 heptyl)amine.
Solubilité mesurée: 500 mg/l.

Exemple 2

Solubilisation directe du trichlorure d'iridium dans le chlorure de méthylène

On opère comme dans l'exemple 1 en utilisant les réactifs suivants:
$Ir\ Cl_3 = 0{,}297$ g (0,001 mole)
Tris(dioxa-3,5 heptyl)amine = 0,32 g (0,001 mole)
$CH_2\ Cl_2 = 20$ cm3
Solubilité mesurée: 9500 mg/l
Solubilité maximum calculée: 9580 mg/l
Taux de solubilisation: 99%
Complexe formé: $[N(CH_2—CH_2—O—CH_2—CH_2—O—CH_3)_3]$ $(Ir\ Cl_3)$
*Essai comparatif:* on opère comme ci-dessus mais sans ajouter de tris(dioxa-3,6 heptyl)amine.
Solubilité mesurée: inférieure à 1 mg/l.

Exemple 3

Solubilisation directe du trichlorure de rhénium dans le chlorure de méthylène

On opère comme dans l'exemple 1 en utilisant les réactifs suivants:
$Re\ Cl_3 = 0{,}292$ g (0,001 mole)
Tris(dioxa-3,6 heptyl)amine = 0,32 g (0,001 mole)
$CH_2\ Cl_2 = 20$ cm3
Solubilité mesurée: 9300 mg/l
Solubilité maximum calculée: 9310 mg/l
Taux de solubilisation: 99%
Complexe formé: $[N(CH_2—CH_2—O—CH_2—CH_2—O—CH_3)_3]$ $(Re\ Cl_3)$
*Essai comparatif:* on opère comme ci-dessus mais sans ajouter de tris(dioxa-3,6 heptyl)amine.
Solubilité mesurée: inférieure à 1 mg/l

Exemple 4

Solubilisation directe du trichlorure de ruthénium dans le chlorure de méthylène

On opère comme dans l'exemple 1 en utilisant les réactifs suivants:
$Ru\ Cl_3 = 0{,}207$ g (0,001 mole)
Tris(dioxa-3,6 heptyl)amine = 0,32 g (0,001 mole)
$CH_2\ Cl_2 = 20$ cm3
Solubilité mesurée: 5000 mg/l
Solubilité maximum calculée: 5050 mg/l
Taux de solubilisation: 99%
Complexe formé: $[N(CH_2—CH_2—O—CH_2—CH_2—O—CH_3)_3]$ $(Ru\ Cl_3)$
*Essai comparatif:* on opère comme ci-dessus mais sans ajouter de tris(dioxa-3,6 heptyl)amine.
Solubilité mesurée: inférieure à 1 mg/l.

Exemple 5

Solubilisation directe du pentachlorure de molybdène dans le chlorure de méthylène

On opère comme dans l'exemple 1 en utilisant les réactifs suivants:
$Mo\ Cl_5 = 0{,}273$ g (0,001 mole)
Tris(dioxa-3,6 heptyl)amine = 0,32 g (0,001 mole)
$CH_2\ Cl_2 = 20$ cm3
Solubilité mesurée: 4250 mg/l
Solubilité maximum calculée: 4800 mg/l
Taux de solubilisation: 88%
Complexe formé: $[N(CH_2—CH_2—O—CH_2—CH_2—O—CH_3)_3]$ $(Mo\ Cl_5)$
*Essai comparatif:* on opère comme ci-dessus mais sans ajouter de tris(dioxa-3,6 heptyl)amine.
Solubilité mesurée: 1360 mg/l.

Exemple 6

Solubilisation directe de trichlorure de rhodium dans le chlorure de méthylène

On opère comme dans l'exemple 1 en utilisant les réactifs suivants:
$Rh\ Cl_3 = 0{,}209$ g (0,001 mole)
Tris(dioxa-3,6 heptyl)amine = 0,32 g (0,001 mole)
$CH_2\ Cl_2 = 20$ cm3
Solubilité mesurée: 5100 mg/l
Solubilité maximum calculée: 5150 mg/l
Taux de solubilisation: 99%
Complexe formé: $[N(CH_2—CH_2—O—CH_2—CH_2—O—CH_3)_3]$ $(Rh\ Cl_3)$
*Essai comparatif:* on opère comme ci-dessus mais sans ajouter de tris(dioxa-3,6 heptyl)amine.
Solubilité mesurée: inférieure à 2 mg/l.

Exemple 7

Solubilisation directe du chlorure de palladium dans le chlorure de méthylène

On opère comme dans l'exemple 1 en utilisant les reactifs suivants:
$Pd\ Cl_2 = 0{,}177$ g (0,001 mole)
Tris(dioxa-3,6 heptyl)amine = 0,32 g (0,001 mole)
$CH_2\ Cl_2 = 20$ cm3
Solubilité mesurée: 2600 mg/l
Solubilité maximum calculée: 5320 mg/l
Taux de solubilisation: 49%
*Essai comparatif:* on opère comme ci-dessus mais sans ajouter de tris(dioxa-3,6 heptyl)amine.
Solubilité mesurée: inférieure à 2 mg/l.

Exemple 8

Solubilisation directe du chlorure de platine dans le chorure de méthylène

On opère comme dans l'exemple 1 en utilisant les réactifs suivants:

$Pt\ Cl_2 = 0,266$ g (0,001 mole)

Tris(dioxa-3,6 heptyl)amine = 0,32 g (0,001 mole)

$CH_2\ Cl_2 = 20$ cm3

Solubilité mesurée: 9700 mg/l

Solubilité maximum calculée: 9755 mg/l

Taux de solubilisation: 99%

Complexe formé: $[N(CH_2—CH_2—O—CH_2—CH_2—O—CH_3)_3]\ (Pt\ Cl_2)$

*Essai comparatif:* on opère comme ci-dessus mais sans ajouter de tris(dioxa-3,6 heptyl)amine.

Solubilité mesurée: inférieure à 1 mg/l.

Exemple 9

Solubilisation directe du pentafluorure de tantale dans le chlorure de méthylène

On opère comme dans l'exemple 1 en utilisant les réactifs suivants:

$Ta\ F_5 = 0,276$ g (0,001 mole)

Tris(dioxa-3,6 heptyl)amine = 0,32 g (0,001 mole)

$CH_2\ Cl_2 = 20$ cm3

Solubilité mesurée: 2420 mg/l

Solubilité maximum calculée: 9050 mg/l

Taux de solubilisation: 26%

*Essai comparatif*: on opère comme ci-dessus mais sans ajouter de tris(dioxa-3,6 heptyl)amine.

Solubilité mesurée: inférieure à 1 mg/l.

Exemple 10

Solubilisation directe du chlorure de fer (ferreux) dans le chlorure de méthylène

On opère comme dans l'exemple 1 en utilisant les réactifs suivants:

$Fe\ Cl_2,\ 4\ H_2O = 0,199$ g (0,001 mole)

Tris(dioxa-3,6 heptyl)amine = 0,32 g (0,001 mole)

$CH_2\ Cl_2 = 20$ cm3

Solubilité mesurée: 950 mg/l

Solubilité maximum calculée: 2794 mg/l

Taux de solubilisation: 34%

*Essai comparatif:* on opère comme ci-dessus mais sans ajouter de tris(dioxa-3,6 heptyl)amine.

Solubilité mesurée: 2 mg/l.

Exemple 11

Solubilisation directe de l'hexacarbonyle molybdène dans le chlorure de méthylène

On opère comme dans l'exemple 1 en utilisant les réactifs suivants:

$Mo\ (CO)_6 = 0,276$ g (0,001 mole)

Tris(dioxa-3,6 heptyl)amine = 0,32 g (0,001 mole)

$CH_2\ Cl_2 = 20$ cm3

Solubilité mesurée: 4780 mg/l

Solubilité maximum calculée: 4797 mg/l

Taux de solubilisation: 99%

*Essai comparatif:* on opère comme ci-dessus mais sans ajouter de tris(dioxa-3,6 heptyl)amine.

Solubilité mesurée: inférieure à 1 mg/l.

Exemple 12

Solubilisation directe du chlorure de titane dans le chlorure de méthylène

On opère comme dans l'exemple 1 en utilisant les réactifs suivants:

$Ti\ Cl_3 = 0,157$ g (0,001 mole)

Tris(dioxa-3,6 heptyl)amine = 0,32 g (0,001 mole)

$CH_2\ Cl_2 = 20$ cm3

Solubilité mesurée: 2390 mg/l

Solubilité maximum calculée: 2395 mg/l

Taux de solubilisation: 99%

Complexe formé: $[N(CH_2—CH_2—O—CH_2—CH_2—O—CH_3)_3]\ (Ti\ Cl_3)$

*Essai comparatif:* on opère comme ci-dessus mais sans ajouter de tris(dioxa-3,6 heptyl)amine.

Solubilité mesurée: inférieure à 1 mg/l.

Exemple 13

Solubilisation directe du perchlorate de magnésium dans le chlorure de méthylène

On opère comme dans l'exemple 1 en utilisant les réactifs suivants:

$Mg(ClO_4)_2$, $H_2O$ = 0,223 g (0,001 mole)
Tris(dioxa-3,6 heptyl)amine = 0,32 g (0,001 mole)
$CH_2Cl_2$ = 20 cm3
Solubilité mesurée: 14 mg/l
Solubilité maximum calculée: 1215 mg/l
Taux de solubilisation: 1%
*Essai comparatif:* on opère comme ci-dessus mais sans ajouter de tris(dioxa-3,6 heptyl)amine.
Solubilité mesurée: inférieure à 0,4 mg/l.

Exemple 14

Solubilisation directe de différents sels de zinc dans le chlorure de méthylène

On opère comme dans l'exemple 1 en utilisant les réactifs suivants:

a) $ZnCl_2$ = 0,136 g (0,001 mole)
Tris(dioxa-3,6 heptyl)amine = 0,32 g (0,001 mole)
$CH_2Cl_2$ = 20 cm3
Solubilité mesurée: 2190 mg/l
Solubilité maximum calculée: 3270 mg/l
Taux de solubilisation: 67%
*Essai comparatif:* on opère comme ci-dessus mais sans ajouter de tris(dioxa-3,6 heptyl)amine.
Solubilité mesurée: inférieure à 1 mg/l.

b) $Zn(NO_3)_2$, 6 $H_2O$ = 0,297 g (0,001 mole)
Tris(dioxa-3,6 heptyl)amine = 0,32 g (0,001 mole)
$CH_2Cl_2$ = 20 cm3
Solubilité mesurée: 81 mg/l
Solubilité maximum calculée: 3270 mg/l
Taux de solubilisation: 2%
*Essai comparatif:* on opère comme ci-dessus mais sans ajouter de tris(dioxa-3,6 heptyl)amine.
Solubilité mesurée: inférieure à 1 mg/l.

Exemple 15

Solubilisation directe de différents sels de nickel dans le chlorure de méthylène

On opère comme dans l'exemple 1 en utilisant les réactifs suivants:

a) $NiCl_2$, 6 $H_2O$ = 0,238 g (0,001 mole)
$CH_2Cl_2$ = 20 cm3
Solubilité mesurée: 800 mg/l
Solubilité maximum calculée: 2935 mg/l
Taux de solubilisation: 27%
*Essai comparatif:* on opère come ci-dessus mais sans ajouter de tris(dioxa-3,6 heptyl)amine.
Solubilité mesurée: inférieure à 1 mg/l.

b) $(CH_3COO)_2Ni$, 4 $H_2O$ = 0,249 g (0,001 mole)
Tris(dioxa-3,6 heptyl)amine = 0,32 g (0,001 mole)
$CH_2Cl_2$ = 20 cm3
Solubilité mesurée: 250 mg/l
Solubilité maximum calculée: 2935 mg/l
Taux de solubilisation: 8,5%
*Essai comparatif:* on opère come ci-dessus mais sans ajouter de tris(dioxa-3,6 heptyl)amine.
Solubilité mesurée: inférieure à 1 mg/l.

Exemple 16

Solubilisation directe de différents sels de cobalt dans le chlorure de méthylène

On opère comme dans l'exemple 1 en utilisant les réactifs suivants:

a) $CoCl_2$, 6 $H_2O$ = 0,238 g (0,001 mole)
Tris(dioxa-3,6 heptyl)amine = 0,32 g (0,001 mole)
$CH_2Cl_2$ = 20 cm3
Solubilité mesurée: 1100 mg/l
Solubilité maximum calculée: 2950 mg/l
Taux de solubilisation: 37%
*Essai comparatif:* on opère comme ci-dessus mais sans ajouter de tris(dioxa-3,6 heptyl)amine.
Solubilité mesurée: inférieure à 1 mg/l

b) $CoBr_2$, 2 $H_2O$ = 0,255 g (0,001 mole)
Tris(dioxa-3,6 heptyl)amine = 0,32 g (0,001 mole)
$CH_2Cl_2$ = 20 cm3
Solubilité mesurée: 800 mg/l
Solubilité maximum calculée: 2950 mg/l
Taux de solubilisation: 27%

*Essai comparatif:* on opère comme ci-dessus mais sans ajouter de tris(dioxa-3,6 heptyl)amine.
Solubilité mesurée: inférieure à 1 mg/l

c) $(CH_3\ COO)_2$ Co, 4 $H_2O$ = 0,242 g (0,001 mole)
Tris(dioxa-3,6 heptyl)amine = 0,32 g (0,001 mole)
$CH_2\ Cl_2$ = 20 cm3
Solubilité mesurée: 150 mg/l
Solubilité maximum calculée: 2950 mg/l
Taux de solubilisation: 5%
*Essai comparatif:* on opère comme ci-dessus mais sans ajouter de tris(dioxa-3,6 heptyl)amine.
Solubilité mesurée: 5 mg/l

Exemple 17

Solubilisation directe de l'acétate de chrome dans le chlorure de méthylène

On opère comme dans l'exemple 1 en utilisant les réactifs suivants:
$(CH_3\ COO)_3$ = 0,229 g (0,001 mole)
Tris(dioxa-3,6 heptyl)amine = 0,32 g (0,001 mole)
$CH_2\ Cl_2$ = 20 cm3
Solubilité mesurée: 67 mg/l
Solubilité maximum calculée: 2600 mg/l
Taux de solubilisation: 2%
*Essai comparatif:* on opère comme ci-dessus mais sans ajouter de tris(dioxa-3,6 heptyl)amine.
Solubilité mesurée: inférieure à 1 mg/l.

Exemple 18

Solubilisation directe du chlorure de mercure dans le chlorure de méthylène

On opère comme dans l'exemple 1 en effectuant deux essais différant par la nature de l'agent séquestrant employé.

*Essai n° 1*
Hg Cl = 0,236 g (0,001 mole)
Tris(dioxa-3,6 heptyl)amine = 0,32 g (0,001 mole)
$CH_2\ Cl_2$ = 20 cm3
Solubilité mesurée: 1200 mg/l
Solubilité maximum calculée: 10020 mg/l
Taux de solubilisation: 12%
*Essai comparatif:* on opère comme ci-dessus mais sans ajouter de tris(dioxa-3,6 heptyl)amine.
Solubilité mesurée: 33 mg/l

*Essai no° 2*
Hg Cl = 0,236 g (0,001 mole)
Tris(trioxa-3,6,9 décyl)amine = 0,455 g (0,001 mole)
$CH_2\ Cl_2$ = 20 cm3
Solubilité mesurée: 9300 mg/l
Solubilité maximum calculée: 10020 mg/l
Taux de solubilisation: 93%
Complexe formé: $[N(CH_2—CH_2—O—CH_2—CH_2—O—CH_3)_3]$ (Hg Cl)
*Essai comparatif:* on opère comme ci-dessus mais sans ajouter de tris(trioxa-3,6,9 décyl)amine.
Solubilité mesurée: 30 mg/l

On constate que la solubilité du chlorure de mercure augmente avec le nombre d'atomes d'oxygène dans la molécule d'agent séquestrant.

Exemple 19

Solubilisation directe du nitrate de mercure dans le chlorure de méthylène

On opère comme dans l'exemple 1 en effectuant deux essais différent par la nature de l'agent séquestrant employé.

*Essai n° 1*
Hg $NO_3$, $H_2O$ = 0,281 g (0,001 mole)
Tris(dioxa-3,6 heptyl)amine = 0,32 g (0,001 mole)
$CH_2\ Cl_2$ = 20 cm3
Solubilité mesurée: 600 mg/l
Solubilité maximum calculée: 10020 mg/l
Taux de solubilisation: 6%
*Essai comparatif:* on opère comme ci-dessus mais sans ajouter de tris(dioxa-3,6 heptyl)amine.
Solubilité mesurée: 33 mg/l

*Essai n° 2*
Hg $NO_3$ = 0,281 g (0,001 mole)
Tris(trioxa-3,6,9 décyl)amine = 0,455 g (0,001 mole)

$CH_2\ Cl_2 = 20$ cm3
Solubilité mesurée: 1200 mg/l
Solubilité maximum calculée: 10020 mg/l
Taux de solubilisation: 12%
*Essai comparatif:* on opère comme ci-dessus mais sans ajouter de tris(trioxa-3,6,9 décyl)amine.
Solubilité mesurée: 30 mg/l

On constate ici aussi que la solubilité augmente avec le nombre d'atomes d'oxygène contenus dans la molécule d'agent séquestrant.

Exemple 20

Solubilisation directe du chlorure de calcium dans le chlorure de méthylène

On opère comme dans l'exemple 1 mais en agitant quelques minutes et en laissant décanter une nuit, en utilisant les réactifs suivants:

$Ca\ Cl_2 = 0{,}111$ g (0,001 mole)
Tris(dioxa-3,6 heptyl)amine = 0,32 g (0,001 mole)
$CH_2\ Cl_2 = 10$ cm3
Solubilité mesurée: 2000 g/l
Solubilité maximum calculée: 4000 mg/l
Taux de solubilisation: 50%
*Essai comparatif:* on opère comme ci-dessus mais sans ajouter de tris(dioxa-3,6 heptyl)amine.
Solubilité mesurée: inférieure à 1 mg/l.

Exemple 21

Solubilisation directe du thiocyanate de cuivre dans le chlorure de méthylène

On opère comme dans l'exemple 1 mais en agitant quelques minutes et en laissant décanter une nuit, en utilisant les réactifs suivants:

Cu SCN = 0,122 g (0,001 mole)
Tris(dioxa-3,6 heptyl)amine = 0,32 g (0,001 mole)
$CH_2\ Cl_2 = 10$ cm3
Solubilité mesurée: 140 mg/l
Solubilité maximum calculée: 6355 mg/l
Taux de solubilisation: 2%
*Essai comparatif:* on opère comme ci-dessus mais sans ajouter de tris(dioxa-3,6 heptyl)amine.
Solubilité mesurée: inférieure à 1 mg/l

Exemple 22

Solubilisation directe du thiocyanate de mercure dans le chlorure de méthylène

On opère comme dans l'exemple 1 mais en agitant quelques minutes et en laissant décanter une nuit, en utilisant les réactifs suivants:

$Hg\ (SCN)_2 = 0{,}316$ g (0,001 mole)
Tris(dioxa-3,6 heptyl)amine = 0,32 g (0,001 mole)
$CH_2\ Cl_2 = 10$ cm3
Solubilité mesurée: 20000 mg/l
Solubilité maximum calculée: 20060 mg/l
Taux de solubilisation: 99%
Complexe formé: $[N(CH_2—CH_2—O—CH_2—CH_2—O—CH_3)_3]\ Hg\ (SCN)_2$
*Essai comparatif:* on opère comme ci-dessus mais sans ajouter de tris(dioxa-3,6 heptyl)amine.
Solubilité mesurée: inférieure à 10 mg/l.

Exemple 23

Solubilisation directe de l'hexachlorure de tungstène dans le toluène

Dans un erlenmeyer de 50 ml équipé d'un réfrigérant ascendant, et d'un agitateur magnétique, on introduit 20 ml de toluène. On ajoute ensuite 0,4 g d'hexachlorure de tungstène (0,001 mole) et 0,365 g de tris(dioxa-3,6 octyl)amine (0,001 mole).

Après avoir agité le mélange pendant 10 mn à la température ambiante, on centrifuge et la solution claire ainsi obtenue est analysée par spectrométrie de flamme.

Solubilité mesurée: 7500 mg/l
Solubilité maximum calculée: 9295 mg/l
Taux de solubilisation: 80%
Complexe formé: $[N(CH_2—CH_2—O—CH_2—CH_2—O—C_2H_5)_3]\ (W\ Cl_6)$
*Essai comparatif:* on opère comme ci-dessus mais sans ajouter de tris(dioxa-3,6 octyl)amine.
Solubilité mesurée: 200 mg/l.

Exemple 24

Solubilisation directe du trichlorure d'iridium dans le toluène

On opère comme dans l'exemple 23 en utilisant les réactifs suivants:

Ir $Cl_3$ = 0,297 g (0,001 mole)

Tris(dioxa-3,6 octyl)amine = 365 g (0,001 mole)

Toluene = 20 cm3

Solubilité mesurée: 2060 mg/l

Solubilité maximum calculée: 9580 mg/l

Taux de solubilisation: 20%

*Essai comparatif:* on opère comme ci-dessus mais sans ajouter de tris(dioxa-3,6 octyl)amine.

Solubilité mesurée: inférieure à 1 mg/l.

Exemple 25

Solubilisation directe du trichlorure de rhénium dans le toluène

On opère comme dans l'exemple 23 en utilisant les réactifs suivants:

Re $Cl_3$ = 0,292 g (0,001 mole)

Tris(dioxa-3,6 octyl)amine = 0,365 g (0,001 mole)

Toluène = 20 cm3

Solubilité mesurée: 2090 mg/l

Solubilité maximum calculée: 9310 mg/l

Taux de solubilisation: 22%

*Essai comparatif:* on opère comme ci-dessus mais sans ajouter de tris(dioxa-3,6 octyl)amine.

Solubilité mesuree: inferieure à 1 mg/l.

Exemple 26

Solubilisation directe du trichlorure de ruthénium dans le toluène

On opère comme dans l'exemple 23 en utilisant les réactifs suivants:

Ru $Cl_3$ = 0,207 g (0,001 mole)

Tris(dioxa-3,6 octyl)amine = 0,365 g (0,001 mole)

Toluène = 20 cm3

Solubilité mesurée: 1600 mg/l

Solubilité maximum calculée: 5050 mg/l

Taux de solubilisation: 32%

Complexe formé: $[N(CH_2—CH_2—O—CH_2—CH_2—O—C_2H_5)_3]$ (Ru $Cl_3$)

*Essai comparatif:* on opère comme ci-dessus mais sans ajouter de tris(dioxa-3,6 octyl)amine.

Solubilite mesurée: inférieure à 1 mg/l.

Exemple 27

Solubilisation directe du pentachlorure de molybdène dans le toluène

On opère comme dans l'exemple 23 en utilisant les réactifs suivants:

Mo $Cl_5$ = 0,273 g (0,001 mole)

Tris(dioxa-3,6 octyl)amine = 0,365 g (0,001 mole)

Toluène = 20 cm3

Solubilité mesurée: 80 mg/l

Solubilité maximum calculée: 4800 mg/l

Taux de solubilisation: 2%

*Essai comparatif*: on opère comme ci-dessus mais sans ajouter de tris(dioxa-3,6 octyl)amine.

Solibilité mesurée: 10 mg/l

Exemple 28

Solubilisation directe du trichlorure de rhodium dans le toluène

On opère comme dans l'exemple 23 en utilisant les réactifs suivants:

Rh $Cl_3$ = 0,209 g (0,001 mole)

Tris(dioxa-3,6 octyl)amine = 0,365 g (0,001 mole)

Toluène = 20 cm3

Solubilité mesurée: 683 mg/l

Solubilité maximum calculée: 5150 mg/l

Taux de solubilisation: 13%

*Essai comparatif*: on opère comme ci-dessus mais sans ájouter de tris(dioxa-3,6 octyl)amine.

Solubilité mesurée: inférieure à 1 mg/l.

Exemple 29

Solubilisation directe du chlorure de platine dans le toluène

On opère comme dans l'exemple 23 en utilisant les réactifs suivants:

Pt $Cl_2$ = 0,266 g (0,001 mole)

Tris(dioxa-3,6 octyl)amine = 0,365 g (0,001 mole)
Toluène = 20 cm3
Solubilité mesurée: 9700 mg/l
Solubilité maximum calculée: 9755 mg/l
Taux de solubilisation: 99%
Complexe formé: $[N(CH_2—CH_2—O—CH_2—CH_2—O—C_2H_5)_3](Pt\ Cl_2)$
*Essai comparatif*: on opère comme ci-dessus mais sans ajouter de tris(dioxa-3,6 octyl)amine.
Solubilité mesurée: inférieure à 1 mg/l.

Exemple 30

Solubilisation directe du trichlorure de ruthénium dans le cyclohexane

Dans un erlenmeyer de 50 ml équipé d'un réfrigérant ascendant et d'un agitateur magnétique, on introduit 20 ml de cyclohexane. On ajoute ensuite 0,207 g de trichlorure de ruthénium (0,001 mole) et 0,365 g de tris(dioxa-3,6 octyl)amine (0,001 mole).

Après avoir agité le mélange pendant 10 mn à la température ambiante, on centrifuge et la solution claire ainsi obtenue est analysée par spectrométrie de flamme.

Solubilité mesurée: 110 mg/l
Solubilité maximum calculée: 5050 mg/l
Taux de solubilisation: 2%
Complexe formé: $[N(CH_2—CH_2—O—CH_2—CH_2—O—C_2H_5)_3](Ru\ Cl_3)$
*Essai comparatif*: on opère comme ci-dessus mais sans ajouter de tris(dioxa-3,6 octyl)amine.
Solubilité mesurée: inférieure à 1 mg/l.

Exemple 31

Solubilisation directe du trichlorure d'iridium dans le cyclohexane

On opère comme dans l'exemple 30 en utilisant les réactifs suivants:
$Ir\ Cl_3$ = 0,299 g (0,001 mole)
Tris(dioxa-3,6 octyl)amine = 0,365 g (0,001 mole)
Cyclohexane = 20 cm3
Solubilité mesurée: 4700 mg/l
Solubilité maximum calculée: 9610 mg/l
Taux de solubilisation: 50%
Complexe formé: $[N(CH_2—CH_2—O—CH_2—CH_2—O—C_2H_5)_3]\ (Ir\ Cl_3)$
*Essai comparatif*: on opère comme ci-dessus mais sans ajouter de tris(dioxa-3,6 octyl)amine.
Solubilité mesurée: inférieure à 1 mg/l.

Exemple 32

Solubilisation directe du chlorure de platine dans le cyclohexane

On opère comme dans l'exemple 30 en utilisant les réactifs suivants:
$Pt\ Cl_2$ = 0,266 g (0,001 mole)
Tris(dioxa-3,6 octyl)amine = 0,365 g (0,001 mole)
Cyclohexane = 20 cm3
Solubilité mesurée: 1500 mg/l
Solubilité maximum calculée: 9755 mg/l
Taux de solubilisation: 15%
Complexe formé $[N(CH_2—CH_2—O—CH_2—CH_2—O—C_2H_5)_3]\ (Pt\ Cl_2)$
*Essai comparatif*: on opère comme ci-dessus mais sans ajouter de tris(dioxa-3,6 octyl)amine.
Solubilité mesurée: inférieure à 1 mg/l.

Exemple 33

Solubilisation directe du trichlorure de rhodium dans le cyclohexane

On opère dans l'exemple 30 en utilisant les réactifs suivants:
$Rh\ Cl_3$ = 0,209 g (0,001 mole)
Tris(dioxa-3,6 octyl)amine = 0,365 g (0,001 mole)
Cyclohexane = 20 cm3
Solubilité mesurée: 20 mg/l
Solubilité maximum calculée: 5150 mg/l
Taux de solubilisation: 0,4%
*Essai comparatif*: on opère comme ci-dessus mais sans ajouter de tris(dioxa-3,6 octyl)amine.
Solubilité mesurée: inférieure à 1 mg/l.

Exemple 34

Solubilisation directe de l'iodure de cadmium dans le chlorure de méthylène

On opère comme dans l'exemple 1 en utilisant les réactifs suivants:
$Cd\ I_2$ = 0,366 g (0,001 mole)

Tris(dioxa-3,6 heptyl)amine = 0,32 g (0,001 mole)
$CH_2\ Cl_2$ = 10 cm3
Solubilité mesurée: 9500 mg/l
Solubilité maximum calculée: 11240 mg/l
Taux de solubilisation: 85%
Complexe formé $[N(CH_2—CH_2—O—CH_2—CH_2—O—CH_3)_3]$ (Cd $I_2$)
*Essai comparatif*: on opère comme ci-dessus mais sans ajouter de tris(dioxa-3,6 heptyl)amine.
Solubilité mesurée: inférieure à 0,5 mg/l.

Exemple 35

Influence de la taille du cation sur le degré de solubilisation dans le chlorure de méthylène

On réalise 3 essais en opérant comme dans l'exemple 1, mais en agitant quelques minutes et en laissant décanter une nuit.

*Essai n° 1*
Li SCN = 0,065 g (0,001 mole)
Tris(dioxa-3,6 heptyl)amine = 0,323 g (0,001 mole)
$CH_2\ Cl_2$ = 10 cm3

*Essai n° 2*
Na SCN = 0,081 g (0,001 mole)
Tris(dioxa-3,6 heptyl)amine = 0,32 g (0,001 mole)
$CH_2\ Cl_2$ = 10 cm3

*Essai n° 3*
K SCN = 0,097 g (0,001 mole)
Tris(dioxa-3,6 heptyl)amine = 0,32 g (0,001 mole)
$CH_2\ Cl_2$ = 10 cm3

On réalise 3 essais comparatifs en opérant comme ci-dessus mais sans ajouter de tris(dicxa-3,6 heptyl)amine.

Les résultats obtenus sont donnés dans le tableau I suivant.

Les complexes formés sont:
*Essai n° 1*: $[N(CH_2—CH_2—O—CH_2—CH_2—O—CH_3)_3]$ (LiSCN)
*Essai n° 2*: $[N(CH_2—CH_2—O—CH_2—CH_2—O—CH_3)_3]$ (Na SCN)
*Essai n° 3*: $[N(CH_2—CH_2—O—CH_2—CH_2—O—CH_3)_3]$ (K SCN)
Le taux de solubilisation sont:
*Essai n° 1*: 98%
*Essai n° 2*: 82%
*Essai n° 3*: 79%

TABLEAU I

| Essai | Sel | Avec tris(dioxa-3,6 heptyl)amine | | Sans tris(dioxa-3,6 heptyl)amine |
|---|---|---|---|---|
| | | Solubilité mesurée | Solubilité maximum calculée | Solubilité mesurée |
| 1 | LiSCN | 680 mg/l | 694 mg/l | <1 mg/l |
| 2 | NaSCN | 1.800 mg/l | 2.300 mg/l | < 1 mg/l |
| 3 | K SCN | 3.100 mg/l | 3.910 mg/l | < 1 mg/l |

Exemple 36

Influence de la température sur le degré de solubilisation du thiocyanate de lithium dans le toluène

10 cm3 d'une solution 0,1 M de tris(dioxa-3,6 heptyl)amine dans du toluène sont agités à température ambiante avec 1,1 millimole de thiocyanate de lithium en poudre.

Après décantation de la solution, on analyse la phase toluène. On constate par analyse infrarouge que la tris(dioxa-3,6 heptyl)amine a disparu preatiquement totalement de la solution. Cela s'explique par la formation d'un complexe:

(Li SCN)[tris(dioxa-3,6 heptyl)amine]

très peu soluble à la température ambiante dans le toluène.

On effectue la même opération mais en agitant à 60°C. La solution devient limpide et on constate par analyse infrarouge que la totalité du complexe précité se trouve dans la phase toluène.

Exemple 37

Solubilisation indirecte du thiocyanate d'ammonium

On dissout 0,455 g (0,001 mole) de tris(trioxa-3,6,9 décyl) amine dans 10 ml de méthanol anhydre puis on introduit 0,076 g (0,001 mole) de thiocyanate d'ammonium. Le mélange est agité pendant 1 heure à la température ambiante. On évapore ensuite lentement le méthanol puis on reprend le mélange obtenu dans 20 cm3 de chlorure de méthylène. On filtre la solution et on évapore le solvant. On obtient un complexe liquide orangé dont la teneur en soufre est de 4,8%, (la teneur maximum calculée est de 6,03%).

Ce complexe répond à la formule:

$$[N(CH_2—CH_2—O—CH_2—CH_2—O—CH_2—CH_2—O—CH_3)_3]\ (NH_4\ SCN).$$

Exemple 38

Solubilisation indirecte du chlorure de lithium dans différents solvants

On dissout 1,6 de tris(dioxa-3,6 heptyl)amine (0,005 mole) dans 30 ml de méthanol anhydre. On introduit dans la solution obtenue 0,22 g (0,005 mole) de chlorure de lithium anhydre. Le mélange est agité pendant 1 heure à température ambiante. On évapore ensuite lentement le méthanol puis on reprend le mélange obtenu dans 20 cm3 de chlorure de méthylène. On filtre la solution et on évapore le solvant. On obtient un liquide orangé qui est le complexe chlorure de lithium-tris(dioxa-3,6 heptyl)amine dont les teneurs en lithium et en chlore sont:

$Li^+$: 1,7% (théorie: 1,91%)

$Cl^-$: 8,6% (théorie: 9,71%)

et dont la formule est $[N(CH_2—CH_2—O—CH_2—CH_2—O—CH_3)_3](Li\ Cl)$

Ce liquide orangé est soluble notamment dans le chlorobenzène, le chloroforme et le diméthylsulfoxyde.

Exemple 39

Solubilisation indirecte du chlorure de lithium

On dissout 0,455 g (0,001 mole) de tris(trioxa-3,6,9 décyl) amine dans 10 ml de mèthanol anhydre puis on introduit 0,042 g (0,001 mole) de chlorure de lithium deshydraté. Le mélange est agité pendant 1 heure à température ambiante. On évapore ensuite lentement le méthanol puis on reprend le mélange obtenu dans 20 cm3 de chlorure de méthylène. On filtre la solution et on évapore le solvant. On obtient un liquide orangé qui est le complexe:

$$[N(CH_2—CH_2—O—CH_2—CH_2—O—CH_2—CH_2—O—CH_3)_3](Li\ Cl)$$

dont les teneurs en lithium et en chlore sont:

$Li^+$: 1,03% (théorie: 1,41%)

$Cl^-$: 5,3% (théorie: 7,16%)

Ce complexe est soluble dans le chlorobenzène, le diméthylsulfoxyde, la N-méthylpyrrolidone, le chloroforme.

Exemple 40

Influence de la teneur en agent séquestrant sur le taux d'extraction du picrate de césium

On prépare une solution aqueuse contenant 0,1 mole/l d'hydroxyde de césium et 0,00115 mole/l d'acide picrique.

On prépare une solution dans le chlorure de méthylène à 0,007 mole/l de tris(dioxa-3,6 heptyl)amine et une solution dans le chlorure de méthylène à 0,007 mole/l de tris(trioxa-3,6,9 décyl)amine.

On mélange des volumes égaux de chacune des solutions d'agents séquestrants avec la solution de picrate de césium.

Dans le cas de la tris(dioxa-3,6 heptyl)amine, on observe une extraction de 22% du picrate de césium.

Dans le cas de tris(trioxa-3,6,9 décyl)amine, on obtient 60%.

Un essai comparatif conduit sans agent séquestrant donne une extraction nulle.

Exemple 41

Influence de la teneur en agent séquestrant sur le taux d'extraction du picrate de sodium

On opère comme suit: on prépare une solution aqueuse A contenant 1 mole/l de soude et 0,00021 mole/l d'acide picrique. On prépare également 9 solutions B d'agents séquestrants selon l'invention dans du chlorure de méthylène en faisant varier la nature de l'agent séquestrant et sa concentration.

On réalise 9 essais en mélangeant des volumes égaux de solutions A et B et en mesurant le pourcentage de picrate de sodium extrait, c'est-à-dire passant de la phase aqueuse dans la phase chlorure de méthylène.

Les résultats obtenus sont donnés dans le tableau II.

TABLEAU II

| Essai | Agent séquestrant | Concentration de l'agent séquestrant mole/l | Picrate de sodium extrait |
|---|---|---|---|
| 1 | tris(dioxa-3,6 heptyl)amine | $8{,}85 \times 10^{-5}$ | 28 % |
| 2 | ,, | $2{,}62 \times 10^{-4}$ | 51 % |
| 3 | ,, | $2{,}6 \times 10^{-3}$ | 92,5 % |
| 4 | tris(dioxa-3,6 octyl)amine | $8{,}08 \times 10^{-5}$ | 11 % |
| 5 | ,, | $2{,}33 \times 10^{-4}$ | 17 % |
| 6 | ,, | $2{,}33 \times 10^{-3}$ | 54 % |
| 7 | tris(dioxa-3,6 décyl)amine | $7{,}01 \times 10^{-5}$ | 8 % |
| 8 | ,, | $2 \times 10^{-4}$ | 12,5 % |
| 9 | ,, | $2 \times 10^{-3}$ | 42,5 % |

On constate que le taux d'extraction augmente avec la quantité d'agent séquestrant utilisée.

On effectue un essai comparatif sans ajouter d'agent séquestrant: le taux d'extraction du picrate de sodium est nul.

Exemple 42

Influence de la nature de l'agent séquestrant sur le taux d'extraction

On opère comme suit: on prépare une solution aqueuse A contenant 0,1 mole/l d'hydroxyde de sodium et 0,0007 mole/l d'acide picrique. On agite pendant 3 heures. On prépare de la même façon 2 autres solutions A en remplaçant l'hydroxyde de sodium par, successivement, l'hydroxyde de potassium et l'hydroxyde de césium.

On prépare, d'autre part, 2 solutions B à 0,0007 mole/l l'une de tris(dioxa-3,6 heptyl)amine, l'autre de tris(trioxa-3,6,9 décyl)amine dans le chlorure de méthylène.

On réalise 6 essais en mélangeant intimement pendant 1 minute des volumes égaux de chacune des 3 solutions A avec chacune des 2 solutions B et en mesurant après décantation le pourcentage de picrate extrait c'est-à-dire passant de la phase aqueuse dans la phase chlorure de méthylène.

Les résultats obtenus sont donnés dans le tableau III.

TABLEAU III

| Sel à extraire | Tris(dioxa-3,6 heptyl) amine | Tris(trioxa-3,6,9 décyl) amine |
|---|---|---|
| Essais 1 et 2<br>$Pi^- Na^+$ | 23% | 13% |
| Essais 3 et 4<br>$Pi^- K^+$ | 18% | 29% |
| Essais 5 et 6<br>$Pi^- Cs^+$ | 7% | 19% |

On réalise parallèlement 3 essais comparatifs en mélangeant les 4 solutions A avec des volumes égaux chlorure de méthylène ne contenant pas d'agent séquestrant: les taux d'extraction des différents picrates sont nuls.

Exemple 43

Extraction du picrate de baryum en solution aqueuse

On opère comme suit: on prépare une solution aqueuse A contenant 0,1 mole/l d'hydroxyde de baryum et 0,014 mole/l d'acide picrique. On agite pendant 3 heures. On prépare d'autre part, 2 solutions B contenant l'une 0,007 mole/l de tris(dioxa-3,6 heptyl)amine, l'autre 0,007 mole/l de tris(trioxa-3,6,9 décyl)amine dans le chlorure de méthylène.

On mélange intimement pendant 1 minute des volumes égaux de la solution A avec chacune des solutions B. On mesure après décantation le pourcentage de picrate extrait, c'est-à-dire passant de la phase aqueuse dans la phase chlorure de méthylène.

Avec la tris(dioxa-3,6 heptyl)amine, on a 75,5% de picrate de baryum extrait; avec la tris(trioxa-3,6,9 décyl)amine, on a 77,5%.

Sans agent séquestrant, le taux d'extraction est nul.

Exemple 44

Solubilisation directe du thiocyanate de plomb dans le méthanol et dans l'acétonitrile

On opère comme dans l'exemple 1 mais en agitant quelques minutes et en laissant décanter une nuit, en effectuant les deux essais suivants:

*Essai n° 1*

$Pb\ (SCN)_2$ = 0,323 g (0,001 mole)
Tris(trioxa-3,6,9 décyl)amine = 0,455 g (0,001 mole)
Méthanol = 10 cm3
Solubilité mesurée: 12900 mg/l
Solubilité maximum calculée: 20720 mg/l
Taux de solubilisation: 62%
*Essai comparatif*: on opère comme ci-dessus mais sans ajouter de tris(trioxa-3,6,9 décyl)amine.
Solubilité mesurée: 250 mg/l

*Essai n° 2*

$Pb\ (SCN)_2$ = 0,323 g (0,001 mole)
Tris(trioxa)3,6,9 décyl)amine = 0,455 g (0,001 mole)
Acétonitrile = 10 cm3
Solubilité mesurée: 500 mg/l
Solubilité maximum calculée: 20720 mg/l
Taux de solubilisation: 2%
Si on laisse décanter 3 jours, on obtient les résultats suivants:
Solubilité mesurée: 2800 mg/l
Taux de solubilisation: 13,5%
*Essai comparatif:* on opère comme ci-dessus mais sans ajouter de tris(trioxa-3,6,9 décyl)amine.
Solubilité mesurée: inférieure à 10 mg/l

Exemple 45

Solubilisation directe de l'acétate de plomb dans l'acétonitrile et dans le chlorure de méthylène

On opère comme dans l'exemple 1 mais en agitant quelques minutes et en laissant décanter une nuit. On effectue les deux essais suivants:

*Essai n° 1*

$(CH_3\ COO)_2\ Pb$ = 0,325 g (0,001 mole)
Tris(trioxa-3,6,9 décyl)amine = 0,455 g (0,001 mole)
Acétonitrile 10 cm3
Solubilité mesurée: 7750 mg/l
Solubilité maximum calculée: 20720 mg/l
Taux de solubilisation: 37,5%
*Essai comparatif*: on opère comme ci-dessus mais sans ajouter de tris(trioxa-3,6,9 décyl)amine.
Solubilité mesurée: 65 mg/l

*Essai n° 2*

$(CH_3\ COO)_2\ Pb$ = 0,325 g (0,001 mole)
Tris(dioxa-3,6 heptyl)amine = 0,32 g (0,001 mole)
Chlorure de méthylène = 10 cm3
Solubilité mesurée: 2000 mg/l

Solubilité maximum calculée: 20720 mg/l
Taux de solubilisation: 10%
*Essai comparatif*: on opère comme ci-dessus mais sans ajouter de tris(dioxa-3,6 heptyl)amine.
Solubilité mesurée: inférieure à 15 mg/l

Exemple 46

Influence de la nature de l'agent séquestrant sur le taux d'extraction du picrate de sodium et de potassium

On opère comme suit: on prépare une solution aqueuse A contenant 1 mole/l de soude et 0,005 mole/l d'acide picrique et une solution aqueuse A' contenant une mole/l de potasse et 0,005 mole/l d'acide picrique. On prépare également 5 solutions B d'agents séquestrants différents à 0,005 mole/l dans du chlorure de méthylène.

On réalise 10 essais en mélangeant 10 cm3 de chacune des solutions B avec 10 cm3 de chacune des solutions A et A' et en mesurant, dans chaque cas les pourcentages de picrate de sodium et de picrate de potassium extraits, c'est-à-dire passant de la phase aqueuse dans la phase chlorure de méthylène.

TABLEAU IV

| Essai | Agent séquestrant | Picrate de sodium extrait (%) | Picrate de potassium extrait (%) |
|---|---|---|---|
| 1– 2 | tris(dioxa-3,6 heptyl) amine | 40,5 | 35 |
| 3– 4 | tris(dioxa-3,6 octyl)amine | 15,5 | 25,5 |
| 5– 6 | tris(trioxa-3,6,9 décyl) amine | 22,5 | 45,5 |
| 7– 8 | tris(tétraoxa-3,6,9,12-tridecyl)amine | 25 | 55 |
| 9–10 | tris(hexaoxa-3,6,9,12,15,18-nonadécyl)amine | 22 | 53 |

Exemple 47

Solubilisation directe de thiocyanates alcalins

On réalise trois essais en opérant comme dans l'exemple 1 en laissant décanter une nuit.

*Essai n° 1*
Li SCN = 0,065 g (0,001 mole)
Tris(oxa-3 butyl)amine = 0,191 g (0,001 mole)
$CH_2Cl_2$ = 10 cm3
Solubilité mesurée: 690 mg/l
Solubilité maximum calculée: 694 mg/l
Taux de solubilisation: 99,5%
*Essai comparatif*: on opère comme ci-dessus mais sans ajouter de tris(oxa-3 butyl)amine.
Solubilité mesurée: <1 mg/l

*Essai n° 2*
Na SCN = 0,081 g (0,001 mole)
Tris(oxa-3 butyl)amine = 0,191 g (0,001 mole)
$CH_2Cl_2$ = 10 cm3
Solubilité mesurée: 2150 mg/l
Solubilité maximum calculée: 2300 mg/l
Taux de solubilisation: 93,5%.
*Essai comparatif*: on opère comme ci-dessus mais sans ajouter de tris(oxa-3 butyl)amine.
Solubilité mesurée: <1 mg/l

*Essai n° 3*
KSCN = 0,097 g (0,001 mole)
Tris(oxa-3 butyl)amine = 0,191 g (0,001 mole)
$CH_2Cl_2$ = 10 cm3

Solubilité mesurée: 490 mg/l
Solubilité maximum calculée: 3910 mg/l
Taux de solubilisation: 12,5%
*Essai comparatif*: on opère comme ci-dessus mais sans ajouter de tris(oxa-3 butyl)amine.
Solubilité mesurée: <1 mg/l.

Exemple n° 48

Solubilisation directe du chlorure de fer dans le chlorure de méthylène

On opère comme dans l'exemple 1 en utilisant les réactifs
$Fe\ Cl_3$: 0,162 g
tris(dioxa-3,6 heptyl)amine: 0,32 g
$CH_2Cl_2$ = 20 cm3
Solubilité mesurée: 2720 mg/l
Solubilité maximum calculée: 2790 mg/l
Taux de solubilisation: 97%
*Essai comparatif*: on opère comme ci-dessus mais sans ajouter de tris(dioxa-3,6 heptyl)amine.
Solubilité mesurée: 1400 mg/l.

Exemple n° 49

Solubilisation directe du chlorure de fer dans le 1,2 dichloroéthane

On opère comme dans l'exemple 1 en utilisant les réactifs:
$Fe\ Cl_3$; 0,162 g
Tris(dioxa-3,6 heptyl)amine: 0,32 g
$C_2H_4Cl_2$: 20 cm3
Solubilité mesurée: 2700 mg/l
Solubilité maximum calculée: 2790 mg/l
Taux de solubilisation: 97%
*Essai comparatif*: on opère comme ci-dessus mais sans ajouter de tris(dioxa-3,6 heptyl)amine.
Solubilité mesurée: 1880 mg/l

Exemple n° 50

Solubilisation directe du chlorure d'antimoine dans le chlorure de méthylène

On opère comme dans l'exemple 1 en utilisant les réactifs:
$SbCl_3$: 0,228 g
tris(oxa-3,6 heptyl)amine: 0,32 g
$CH_2Cl_2$: 20 cm3
Solubilité mesurée: 5950 mg/l
Solubilité maximum calculée: 6090 mg/l
Taux de solubilisation: 98%
*Essai comparatif*: on opère comme ci-dessus mais sans ajouter de tris(dioxa-3,6 heptyl)amine.
Solubilité mesurée: 5650 mg/l.

Exemple n° 51

Solubilisation directe du méthoxyphénate de sodium dans le chlorure de méthylène

On opére comme dans l'exemple 1 en utilisant les réactifs:
Méthoxyphénate de sodium = 0,146 g
Tris(dioxa-3,6 heptyl)amine = 0,32 g
$CH_2Cl_2$ = 10 cm3
Solubilité mesurée: 380 mg/l
Solubilité maximum calculée: 1150 mg/l
Taux de solubilisation: 33%
*Essai comparatif*: on opère comme ci-dessus mais sans ajouter de tris(dioxa-3,6 heptyl)amine.
Solubilité mesurée: <10 mg/l

Exemple n° 52

Solubilisation directe du nitrate de lanthane dans le chlorure de méthylène

Dans un erlenmeyer de 50 ml équipé d'un réfrigérant ascendant et d'un agitateur magnétique, on introduit 20 ml de chlorure de méthylène anhydre et purifié. On ajoute 1/1000 de mole de sel et 1/1000 de mole de tris(dioxa-3,6 heptyl)amine.

Après agitation pendant 1 heure à la température ambiante, on centrifuge et la solution claire ainsi obtenue est analysée par fluorescence X.
$La(NO_3)_3$, $6H_2O$ = 0,433 g
Tris(dioxa-3,6 heptyl)amine = 0,32 g
$CH_2Cl_2$ = 20 cm3
Solubilité mesurée: 1440 mg/l

Solubilité maximum calculée: 6950 mg/l
Taux de solubilisation: 21%
*Essai comparatif*: on opère comme ci-dessus mais sans ajouter de tris(dioxa-3,6 heptyl)amine.
Solubilité mesurée: <10 mg/l

Exemple n° 53

Solubilisation directe du nitrate de cérium dans le chlorure de méthylène

On opère comme dans l'exemple 52 en utilisant les réactifs
$Ce(NO_3)_3$, $6H_2O$ = 0,434 g
Tris(dioxa-3,6 heptyl)amine = 0,32 g
$CH_2Cl_2$ = 20 cm3
Solubilité mesurée: 1740 mg/l
Solubilité maximum calculée: 7010 mg/l
Taux de solubilisation: 25%
*Essai comparatif*: on opère comme ci-dessus mais sans ajouter de tris(dioxa-3,6 heptyl)amine.
Solubilité mesurée: <10 mg/l

Exemple n° 54

Solubilisation directe du nitrate d'Europium dans le chlorure de méthylène.

On opère comme dans l'exemple 52 en utilisant les réactifs
$Eu(NO_3)_3$, $6H_2O$ = 0,446 g
Tris(dioxa-3,6 heptyl)amine = 0,32 g
$CH_2Cl_2$ = 20 cm3
Solubilité mesurée: 250 mg/l
Solubilité maximum calculée: 7600 mg/l
Taux de solubilisation: 3%
*Essai comparatif*: on opère comme ci-dessus mais sans ajouter de tris(dioxa-3,6 heptyl)amine.
Solubilité mesurée: 50 mg/l.

Exemple n° 55

Solubilisation directe du nitrate de thorium dans le chlorure de méthylène.

On opère comme dans l'exemple 52 en utilisant les réactifs:
$Th(NO_3)_4$, 4 $H_2O$ = 0,552 g
Tris(dioxa-3,6 heptyl)amine: 0,32 g
$CH_2Cl_2$ = 20 cm3
Solubilité mesurée: 5500 mg/l
Solubilité maximum calculée: 11600 mg/l
Taux de solubilisation: 47%
*Essai comparatif*: on opère comme ci-dessus mais sans ajouter de tris(dioxa-3,6 heptyl)amine.
Solubilité mesurée: <10 mg/l

Exemple n° 56

Solubilisation directe du nitrate d'uranyle dans le chorure de méthylène.

On opère comme dans l'exemple 52 en utilisant les réactifs:
$UO_2(NO_3)_2$, $6H_2O$ = 0,502 g
Tris(dioxa-3,6 heptyl)amine = 0,32 g
$CH_2Cl_2$ = 20 cm3
Solubilité mesurée: 6100 mg/l
Solubilité maximum calculée: 11900 mg/l
Taux de solubilisation: 51%
*Essai comparatif*: on opère comme ci-dessus mais sans ajouter de tris(dioxa-3,6 heptyl)amine.
Solubilité mesurée: <10 mg/l.

Exemples 57 à 62

Preparation des agents séquestrants

Les exemples 57 à 62 décrivent la préparation de plusieurs agents séquestrants. Tous les autres agents séquestrants visés par la présente invention peuvent être préparés par des procédés analogues.

Exemple 57

Préparation de la tris(dioxa-3,6 heptyl)amine — Préparation du méthoxy-2 éthanolate de sodium

Dans un ballon tricol d'un litre, muni d'un agitateur mécanique, d'un thermomètre, d'un réfrigérant, on introduit 380 g de méthoxy-2 éthanol (5 moles). On ajoute 23 g de sodium (1 mole) en 3 heures en maintenant la température de mélange à 40°C.

— Synthèse de la tris(dioxa-3,6 heptyl)amine

Au mélange précédent, on ajout 51,6 g de chlorhydrate de tris(chloro-2 éthyl)amine (soit 0,215 mole). On chauffe alors le mélange à reflux du méthoxy-2 éthanol (125°C) pendant 12 heures, puis on distille le solvant sous pression réduite. Le méthoxy-2 éthanolate de sodium en excès est neutralisé par addition de 11,6 cm3 d'HCl aqueux (10 N). Le chlorure de sodium est filtré et la solution est distillée.

La tris(dioxa-3,6 heptyl)amine distille entre 165°C et 180°C sous 0,5 mmHg. On obtient 49 g de produit soit un rendement de 70%.

Exemple 58

Préparation de la tris(dioxa-3,6 décyl)amine

Dans un ballon tricol de 1 litre décrit à l'exemple 1, on introduit 590 g de oxa-3 heptanol-1 (monoéther butylique de l'éthylène glycol). On ajoute 40 g de soude en pastille puis on chauffe le mélange à 120°C. Il se forme alors l'oxa-3 heptanolate de sodium et de l'eau qui distille.

Lorsque toute l'eau de la réaction est éliminée, on introduit 55 g de chlorhydrate de tris(chloro-2 éthyl)amine. Le mélange est chauffé à 130°C pendant 5 heures puis refroidi et l'excès d'alcoolate de sodium neutralisé par une solution aqueuse d'acide chlorhydrique à 10%. Après élimination de l'oxa-3 heptanol par distillation et du chlorure de sodium par filtration, la tris(dioxa-3,6 décyl)amine est distillée (192°C sous 0,1 mmHg).

Exemple 59

Préparation de la tris(trioxa-3,6,9 décyl)amine.

Dans un ballon tricol de 1 litre équipé d'un agitateur mécanique, d'un condenseur et d'un thermomètre, on introduit 600 g d'éther monométhylique du diéthylène glycol (dioxa-3,6 heptanol-1) soit 5 moles puis 23 g de sodium (1 mole) par petites fractions afin de former le dioxa-3,6 heptanolate de sodium.

Lorsque le sodium est totalement transformé, on ajoute alors 51,8 g de chlorhydrate de la tris(chloro-2 éthyl)amine (soit 0,215 mole). Le mélange est chauffé à 130°C pendant 8 heures sous agitation puis refroidi et l'excès d'alcoolate de sodium neutralisé par une solution aqueuse d'acide chlorhydrique à 10%. Le dioxa-3,6 heptanol-1 est éliminé par distillation à 130°C sous 20 mmHg. Le mélange obtenu est filtré afin d'éliminer le chlorure de sodium puis le produit est distillé. On obtient ainsi 83 g de tris(trioxa-3,6,9 décyl)amine qui distille à 189°C sous 0,1 mmHg.

Exemple 60

Préparation de la tris(oxa-3 butyl)amine

Dans un ballon de 1 litre tricol équipé d'une agitation, d'un thermomètre et d'une colonne à distiller, on dissout 1 atome gramme de sodium: 23 g dans 244 g de méthanol. On ajoute ensuite une solution de 30 g soit 0,125 mole de chlorhydrate de tris(chloréthyl)amine dans 150 g de méthanol, puis on maintient la masse au reflux pendant 4 heures.

Après refroidissement, on neutralise le méthylate en excès par 75 g d'HCl concentré. On concentre le méthanol et on extrait la couche aqueuse restante par 2 fois 100 g de dichlorométhane.

Après évaporation du dichlorométhane, on distille la tris(oxa-3-butyl)amine pour obtenir 18 g d'amine tertiaire attendue. Pt $Eb_1$: 70°.

Le rendement sur le chlorhydrate de tris(chloréthyl)amine est de 75,7%.

Exemple 61

Préparation de la tris(tetraoxa-3,6,9,12 tridécyl)amine

Dans un ballon de 3 litres tricol équipé d'une agitation, d'un thermomètre, d'une ampoule de coulée et d'une colonne à reflux, on dissout 115 g de sodium (5 moles) dans 1640 g d'éther monométhylique du triéthylène glycol (10 moles). La suspension obtenue est maintenue à 80° tandis que l'on coule 241 g (1 mole) de chlorhydrate de tris (βchloroéthyl)amine en solution dans 492 g d'éther monométhylique du triéthylène glycol.

La précipitation de NaCl est lente et on maintient la masse réactionnelle à 120—130° pendant 12 heures.

On distille alors sous vide pour éliminer la majeure partie de l'éther monométhylique du triéthylène glycol.

Le résidu après refroidissement est repris par 2000 ml du dichlorométhane acidifié, puis filtré pour éliminer le chlorure de sodium. Le filtrat est alors soumis à distillation pour éliminer le dichlorométhane et l'éther monométhylique du triéthylène glycol. L'amine tertiaire reste en pied de distillation et elle est purifiée par passage sur une colonne de silice.

On obtient 437 g de produit, soit un rendement de 74,7% par rapport au chlorhydrate de tris(βchloréthyl)amine.

Exemple 62

Préparation de la tris(hexaoxa 3,6,9,12,15,18 nona décyl)amine

Dans l'appareillage de l'exemple 61, on dissout 3 moles de sodium soit 69 g dans 1500 g d'éther

monométhylique du pentaéthylène glycol vers 80°C puis on ajoute à la suspension obtenue 120 g (0,5 mole) de chlorhydrate de tris(βchloroéthyl)amine dissous dans 600 g d'éther monométhylique du pentaéthylène glycol. La réaction est lente et nécessite un chauffage de 15 heures à 125—140°.

Après refroidissement, on élimine de chlorure de sodium par filtration et on étête l'éther monométhylique du pentaéthylèneglycol jusqu'à 300° sous 0,5 mmHg.

L'amine tertiaire obtenue est de nouveau filtrée puis passée sur une colonne de silice.

Ob obtient finalement 331 g d'amine tertiaire cherchée soit 78% de rendement par rapport au chlorhydrate engagé de tris(β-chloroéthyl)amine.

**Revendications pour les états contractants BE, CH, DE, FR, GB, IT, LU, NL, SE**

1. Procédé pour solubiliser un sel organique ou minéral dans un solvant organique dans lequel le sel organique ou minéral n'est initialement pas soluble ou pour augmenter la solubilité d'un sel organique ou minéral dans un solvant organique, caractérisé en ce que l'on met en présence le sel organique ou minéral de formule $A^-M^+$ dans laquelle $A^-$ représente un anion minéral ou organique et $M^+$ représente un cation choisi parmi le groupe comprenant le cation $NH_4{}^+$ et ses dérivés et les cations dérivés des métaux des groupes $I_A$, $II_A$, $III_A$, $IV_A$, $V_A$, $VI_A$, $VII_A$, VIII, $I_B$, $II_B$, $III_B$, $IV_B$ et $V_B$ de la classification périodique des éléments et au moins un agent séquestrant, soluble dans ledit solvant organique, de formule: $N—[CHR_1—CHR_2—O—(CHR_3—CHR_4—O)_n—R_5]_3$ (I) dans laquelle n est un nombre entier supérieur ou égal à 0 et inférieur ou égal à 10 ($0 \leqslant n \leqslant 10$), $R_1$ $R_2$ $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $—C_mH_{2m}— \phi$ ou $C_mH_{2m+1}—\phi—$, où m est compris entre 1 et 12, l'agent séquestrant de formule (I) et le sel organique ou minéral formant un complexe de formule: $[N—[CHR_1—CHR_2—O—(CHR_3—CHR_4—O—)_nR_5]_3]_y(M^+A^-)$ (II) dans laquelle y est supérieur ou égal à 1 et inférieur ou égal à 3 ($1 \leqslant y \leqslant 3$), ce complexe de formule (II) étant soluble dans ledit solvant organique.

2. Procédé selon la revendication 1 caractérisé en ce que l'on met en présence en une seule étape le sel organique ou minéral anhydre et l'agent séquestrant de formule (I) en solution dans ledit solvant organique.

3. Procédé selon la revendication 1 caractérisé en ce que l'on met en présence en une seule étape le sel organique ou minéral en solution aqueuse et l'agent séquestrant de formule (I) en solution dans ledit solvant organique.

4. Procédé selon la revendication 1 caractérisé en ce que l'on met en présence, dans une première étape, le sel minéral ou organique en solution dans un tiers solvant et l'agent séquestrant de formule (I) en solution dans le même tiers solvant, en ce que l'on élimine, dans une deuxième étape, le tiers solvant et en ce que l'on met en présence, dans une troisième étape, le produit résultant de la deuxième étape et ledit solvant.

5. Procédé selon la revendication 1, caractérisé en ce que l'on met en présence, dans une première étape, le sel minéral ou organique anhydre et l'agent séquestrant de formule (I) en l'absence de tout solvant et en ce que l'on met en présence, dans une deuxième étape, le produit résultant de la première étape et ledit solvant organique.

6. Procédé selon l'une quelconque des revendications précédentes caractérisé en ce que dans la formule (I), $R_1$ $R_2$ $R_3$ et $R_4$ identiques ou différents représentent un atome d'hydrogène ou un radical méthyle.

7. Procédé selon l'une quelconque des revendications 1 à 5 caractérisé en ce que dans la formule (I) n est un nombre entier supérieur ou égal à 0 et inférieur ou égal à 6.

8. Procédé selon l'une quelconque des revendications 1 à 5 caractérisé en ce que dans la formule (I) $R_5$ représente un radical alkyle ayant de 1 à 4 atomes de carbone.

9. Procédé selon l'une quelconque des revendications 1 à 5 caractérisé en ce que dans la formule (I) $R_1$ $R_2$ $R_3$ et $R_4$ identiques ou différents représentent un atome d'hydrogène ou un radical méthyle, n est un nombre entier supérieur ou égal à 0 et inférieur ou égal à 6 et $R_5$ représente un radical alkyle ayant de 1 à 4 atomes de carbone.

10. Procédé selon la revendication 9 caractérisé en ce que l'agent séquestrant de formule (I) est la tris(dioxa-3,6 heptyl) amine de formule:

$$N—(CH_2—CH_2—O—CH_2—CH_2—O—CH_3)_3$$

11. Procédé selon la revendication 9 caractérisé en ce que l'agent séquestrant de formule (I) est la tris(trioxa-3,6,9-décyl)amine de formule:

$$N—(CH_2—CH_2—O—CH_2—CH_2—O—CH_2—CH_2—O—CH_3)_3$$

12. Procédé selon la revendication 9 caractérisé en ce que l'agent séquestrant de formule (I) est la tris(dioxa-3,6-octyl) amine de formule:

$$N—(CH_2—CH_2—O—CH_2—CH_2—O—C_2H_5)_3$$

13. Procédé selon la revendication 9 caractérisé en ce que l'agent séquestrant de formule (I) est la tris(trioxa-3,6,9-undécyl)amine de formule:

$$N—(CH_2—CH_2—O—CH_2—CH_2—O—CH_2—CH_2—O—C_2H_5)_3$$

14. Procédé selon la revendication 9 caractérisé en ce que l'agent séquestrant de formule (I) est la tris(dioxa-3,6 nonyl) amine de formule:

$$N—(CH_2—CH_2—O—CH_2—CH_2—O—C_3H_7)_3$$

15. Procédé selon la revendication 9 caractérisé en ce que l'agent séquestrant de formule (I) est la tris(trioxa-3,6,9 dodécyl)amine de formule:

$$N—(CH_2—CH_2—O—CH_2—CH_2—O—CH_2—CH_2—O—C_3H_7)_3$$

16. Procédé selon la revendication 9 caractérisé en ce que l'agent séquestrant de formule (I) est la tris(dioxa-3,6 décyl) amine de formule:

$$N—(CH_2—CH_2—O—CH_2—CH_2—O—C_4H_9)_3$$

17. Procédé selon la revendication 9 caractérisé en ce que l'agent séquestrant de formule (I) est la tris(trioxa-3,6,9 tridécyl)amine de formule:

$$N—(CH_2—CH_2—O—CH_2—CH_2—O—CH_2—CH_2—O—C_4H_9)_3$$

18. Procédé selon la revendication 9 caractérisé en ce que l'agent séquestrant de formule (I) est la tris(oxa-3 butyl) amine de formule:

$$N—(CH_2—CH_2—O—CH_3)_3$$

19. Procédé selon la revendication 9 caractérisé en ce que l'agent séquestrant de formule (I) est la tris(tétraoxa-3,6,9,12 tridécyl) amine de formule:

$$N—(CH_2—CH_2—O—CH_2—CH_2—O—CH_2—CH_2—O—CH_2—CH_2—O—CH_3)_3$$

20. Procédé selon la revendication 9 caractérisé en ce que l'agent séquestrant de formule (I) est la tris(hexaoxa-3,6,9,12,15,18 nonadécyl) amine de formule:

$$[N—[(CH_2—CH_2—O)_6—CH_3]_3]_y$$

21. Procédé selon la revendication 9 caractérisé en ce que l'agent séquestrant de formule (I) est la tris(dioxa-3,6 methyl-4 heptyl) amine de formule:

$$N—(CH_2—CH_2—O—CHCH_3—CH_2—O—CH_3)_3$$

22. Procédé selon la revendication 9 caractérisé en ce que l'agent séquestrant de formule (I) est la tris(dioxa-3,6 diméthyl-2,4 heptyl) amine de formule:

$$N—(CH_2—CHCH_3—O—CHCH_3—CH_2—O—CH_3)_3$$

23. Procédé selon la revendication 1 caractérisé en ce que le cation $M^+$ est choisi parmi le groupe comprenant le cation $NH_4^+$, les cations $RNH_3^+$ où R est un radical alkyle ou aryle et les cations dérivés des métaux Li, Na, K, Rb, Cs, Mg, Ca, Sr, Ba, Sc, Y, La et lanthanides, Ac et actinides, Ti, Zr, Hf, V, Nb, Ta, Cr, Mo, W, Mn, Tc, Re, Fe, Co, Ni, Ru, Rh, Pd, Os, Ir, Pt, Cu, Ag, Au, Zn, Cd, Hg, Al, Ga, In, Tl, Ge, Sn, Pb, Sb, Bi.

24. Procédé selon la revendication 1 caractérisé en ce que l'anion $A^-$ est choisi parmi le groupe comprenant

$SCN^-$, $O{=}C{=}N^-$, $Cl^-$, $Br^-$, $I^-$, $F^-$, $CN^-$, $SH^-$, $S^=$, $OH^-$, $HSO_3^-$, $ClO_4^-$, $BrO_4^-$, $NH_2^-$,

$NO_3^-$, $NO_2^-$, $BF_4^-$, $BrO^-$, $ClO^-$, $BH_4^-$, $SO_3^{--}$, $PO_4^{3-}$, $CO_3^{--}$, $SO_4^{--}$, $ClO_3^-$, $BrO_3^-$, $H^-$, $AlH_4^-$.

25. Procédé selon la revendication 1 caractérisé en ce que l'anion $A^-$ est choisi parmi le groupe

comprenant les anions dérivés des alcools, des phenols, des thiols, des thiophénols, des acides, des amines, des amides, des composés organiques à hydrogène mobile et des silanols.

26. Procédé selon la revendication 1 caractérisé en ce que ledit solvant organique est choisi parmi le groupe comprenant l'hexane, le cyclohexane, le benzène, le toluène, l'o-xylène, le m-xylène, le p-xylène, le chlorobenzène, l'o-dichlorobenzène le trichloro-1,2,4 benzène, le chloroforme, le chlorure de méthylène, le tétrachlorure de carbone, le dichloro-1,2 éthane, le trichloro-1,1,1 éthane, le trichloro-1,1,2 éthane, le trichloro-1,2,2 trifluoro-1,1,2 éthane, le perchloroéthylène l'acétonitrile, la diméthylformamide, le N-méthylpyrrolidone, la diméthylacétamide, l'hexaméthylphosphorotriamide, le diméthylsulfoxyde, le sulfolane, le méthanol; l'éthanol et l'isopropanol.

27. Procédé selon la revendication 1 caractérisé en ce qu'on opère à une température comprise entre −50°C et 250°C.

28. Procédé selon la revendication 1 caractérisé en ce qu'on utilise l'agent séquestrant de formule (I) en quantité telle que le rapport molaire de l'agent séquestrant au sel $A^- M^+$ est compris entre 0,001 et 50.

29. Application du procédé selon l'une quelconque des revendications précédentes à la mise en oeuvre du sel $A^- M^+$ en solution dans ledit solvant organique, dans une réaction de sythèse organique.

30. Application de procédé selon l'une quelconque des revendications 1 à 28 à l'extraction du sel $A^- M^+$ d'une solution le contenant.

31. Complexe de formule:

$$[N[CHR_1—CHR_2—O—(CHR_3—CHR_4—O)_n\ R_5]_3]_y\ (M^+\ A^-) \qquad (II)$$

dans laquelle y est supérieur ou égal à 1 et inférieur ou égal à 3 ($1 \leqslant y \leqslant 3$) et dans laquelle n est un nombre entier supérieur ou égal à 0 et inférieur ou égal à 10 ($0 \leqslant n \leqslant 10$), $R_1$ $R_2$ $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $—C_m H_{2m}—\phi$ ou $C_m— H_{2m+1}—\phi—$ ($1 \leqslant m \leqslant 12$), $A^-$ représentant un anion minéral ou organique et $M^+$ représentant un cation choisi parmi le groupe comprenant le cation $NH_4^+$ et ses dérivés et les cations dérivés des métaux des groupes $I_A$, $II_A$, $III_A$, $IV_A$, $V_A$, $VI_A$, $VII_A$, VIII, $I_B$, $II_B$, $III_B$, $IV_B$ et $V_B$ de la classification périodique des éléments.

32. Complexe selon la revendication 31 caractérisé en ce que dans la formule II, $R_1$ $R_2$ $R_3$ et $R_4$ identiques ou différents représentent un atome d'hydrogène ou un radical méthyle.

33. Complexe selon la revendication 31 caractérisé en ce que dans la formule II, n est un nombre entier supérieur ou égal à 0 et inférieur ou égal à 6.

34. Complexe selon la revendication 31 caractérisé en ce que dans la formule II, $R_5$ représente un radical alkyle ayant de 1 à 4 atomes de carbone.

35. Complexe selon la revendication 31 caractérisé en ce que dans la formule II, $R_1$ $R_2$ $R_3$ et $R_4$ identiques ou différents représentent un atome d'hydrogène ou un radical méthyle, n est un nombre entier supérieur ou égal à 0 et inférieur ou égal à 6 et $R_5$ représente un radical alkyle ayant de 1 à 4 atomes de carbone.

36. Complexe selon la revendication 33 caractérisé en ce qu'il répond à la formule:

$$[N—(CH_2—CH_2—O—CH_2—CH_2—O—CH_3)_3]_y\ (A^-M^+)$$

37. Complexe selon la revendication 35 caractérisé en ce qu'il répond à la formule:

$$[N—(CH_2—CH_2—O—CH_2—CH_2—O—CH_2—CH_2—O—CH_3)_3]_y\ (A^-M^+)$$

38. Complexe selon la revendication 35 caractérisé en ce qu'il répond à la formule:

$$[N—(CH_2—CH_2—O—CH_2—CH_2—O—C_2H_5)_3]_y\ (A^-M^+)$$

39. Complexe selon la revendication 35 caractérisé en ce qu'il répond à la formule:

$$[N—(CH_2—CH_2—O—CH_2—CH_2—O—CH_2—CH_2—O—C_2H_5)_3]_y\ (A^-M^+)$$

40. Complexe selon la revendication 35 caractérisé en ce qu'il répond à la formule:

$$[N—(CH_2—CH_2—O—CH_2—CH_2—O—C_3H_7)_3]_y\ (A^-M^+)$$

41. Complexe selon la revendication 35 caractérisé en ce qu'il répond à la formule:

$$[N—(CH_2—CH_2—O—CH_2—CH_2—O—CH_2—CH_2—O—C_3H_7)_3]_y\ (A^-M^+)$$

42. Complexe selon la revendication 35 caracterisé en ce qu'il répond à la formule:

$$[N—(CH_2—CH_2—O—CH_2—CH_2—O—C_4H_9)_3]_y\ (A^-M^+)$$

43. Complexe selon la revendication 35 caractérise en ce qu'il répond à la formule:

$$[N—(CH_2—CH_2—O—CH_2—CH_2—O—CH_2—CH_2—O—C_4H_9)_3]_y\ (A^-M^+)$$

44. Complexe selon la revendication 35 caractérisé en ce qu'il répond à la formule:

$$[N—(CH_2—CH_2—O—CH_3)_3]_y\ (A^-M^+)$$

45. Complexe selon la revendication 35 caractérisé en ce qu'il répond à la formule:

$$[N—(CH_2—CH_2—O—CH_2—CH_2—O—CH_2—CH_2—O—CH_2—CH_2—O—CH_3)_3]_y\ (A^-M^+)$$

46. Complexe selon la revendication 35 caractérisé en ce qu'il répond à la formule:

$$[N—[(CH_2—CH_2—O)_6—CH_3]_3]_y\ (A^-M^+)$$

47. Complexe selon la revendication 35 caractérisé en ce qu'il répond à la formule:

$$[N—(CH_2—CH_2—O—CH_2—CH_2—O—CH_2—CH_2—O—C_4H_9)_3]_y\ (A^-M^+)$$

48. Complexe selon la revendication 35 caractérisé en ce qu'il répond à la formulé:

$$[N—(CH_2—CH_2—O—CH_3)_3]_y\ (A^-M^+)$$

49. Complexe selon l'une quelconque des revendications 31 à 48 caractérisé en ce que dans la formule II, le cation $M^+$ est choisi parmi le groupe comprenant le cation $NH_4^+$, les cations $RNH_3^+$ où R est un radical alkyle ou aryle et les cations dérivés des métaux Li, Na, K, Rb, Cs, Mg, Ca, Sr, Ba, Sc, Y, La et lanthanides, Ac et actinides, Ti, Zr, Hf, V, Nb, Ta, Cr, Mo, W, Mn, Tc, Re, Fe, Co, Ni, Ru, Rh, Pd, Os, Ir, Pt, Cu, Ag, Au, Zn, Cd, Hg, Al, Ga, In, Tl, Ge, Sn, Pb, Sb, Bi.

50. Complexe selon l'une quelconque des revendications 31 à 49 caractérisé en ce que dans la formule II, l'anion $A^-$ est choisi parmi le groupe comprenant

$$SCN^-,\ O{=}C{=}N^-,\ Cl^-,\ Br^-,\ I^-,\ F^-,\ CN^-,\ SH^-,\ S^=,\ OH^-,\ HSO_3^-,\ ClO_4^-,\ BrO_4^-,\ NH_2^-,$$

$$NO_3^-,\ NO_2^-,\ BF_4^-,\ H^-,\ BrO^-,\ ClO^-,\ BH_4^-,\ SO_3^{--},\ PO_4^{3-},\ CO_3^{--},\ SO_4^{--},\ ClO_3^-,\ BrO_3^-,\ AlH_4^-.$$

51. Complexe selon l'une quelconque des revendications 31 à 49 caractérisé en ce que dans la formule II, l'anion $A^-$ est choisi parmi le groupe comprenant les anions dérivés des alcools, des phénols, des thiols, des thiophénols, des acides, des amines, des amides, des composés organiques à hydrogène mobile et des silanols.

**Patentansprüche für die Vertragsstaaten: BE, CH, DE, FR, GB, IT, LU, NL, SE**

1. Verfahren zum Lösen eines organischen- oder Mineralsalzes in einem organischen Lösungsmittel, in dem das organische- oder Mineralsalz nicht unmittelbar löslich ist oder um die Löslichkeit eines organischen- oder Mineralsalzes in einem organischen Lösungsmittel zu steigern, dadurch gekennzeichnet, daß man das organische- oder Mineralsalz der Formel $A^-M^+$, in der $A^-$ ein mineralisches oder organisches Anion und $M^+$ ein Kation darstellt, das aus der Gruppe ausgewählt ist, die das Kation $NH_4^+$ und seine Derivate sowie die Kationen der Metalle der Gruppen $I_A$, $II_A$, $III_A$, $IV_A$, $V_A$, $VI_A$, $VII_A$, VIII, $I_B$, $II_B$, $III_B$, $IV_B$ und $V_B$ des Periodensystems umfasst und mindestens ein Sequestrierungsmittel einsetzt, das in dem genannten organischen Lösungsmittel löslich ist, mit der Formel $N—[CHR_1—CHR_2—O—(CHR_3—CHR_4—O)_n—R_5]_3$ (I) in der n eine ganze Zahl größer oder gleich 0 und kleiner oder gleich 10 ist ($0 \leqslant n \leqslant 10$), $R_1$, $R_2$, $R_3$ und $R_4$ gleich oder unterschiedlich ein Wasserstoffatom oder einen Alkylrest mit 1 bis 4 Kohlenstoffatomen darstellen und $R_5$ ein Alkylrest oder Cycloalkylrest mit 1 bis 12 Kohlenstoffatomen, ein Phenylrest oder ein Radikal $—C_m H_{2m}—\phi$ oder $C_m H_{2m+1}—\phi—$ ist, wo m zwischen 1 und 12 liegt und das Sequestrierungsmittel der Formel (I) und das organische- oder Mineralsalz einen Komplex der Formel:

$$[N—[CHR_1—CHR_2—O—(CHR_3—CHR_4—O—)_nR_5]_3]_y(MA^-) \qquad (II)$$

bilden, in der y größer oder gleich 1 und kleiner oder gleich 3 ist ($1 \leqslant y \leqslant 3$), wobei dieser Komplex der Formel (II) in dem genannten organischen Lösungsmittel löslich ist.

...n gekennzeichnet, daß man das wasserfreie organ... ...genannten organischen Lösungsmittel in Lösung vorliegende Se... ...el (I) in einer einzigen Verfahrensstufe einsetzt.

...n nach Anspruch 1, dadurch gekennzeichnet, daß man das organische- oder Minera... ...wässriger Lösung und das in dem genannten organischen Lösungsmittel in Lösung vorliegend... Sequestrierungsmittel der Formel (I) in einer einzigen Verfahrensstufe einsetzt.

4. Verfahren nach Anspruch 1, dadurch gekennzeichnet, daß man in einer ersten Stufe das in einem Lösungsvermittler gelöste Mineral- oder organische Salz und das in dem selben Lösungsvermittler gelöste Sequestrierungsmittel der Formel (I) einsetzt, daß man in einer zweiten Stufe den Lösungsvermittler beseitigt und daß man in einer dritten Stufe das aus der zweiten Stufe erhaltene Produkt und das genannten Lösungsmittel einsetzt.

5. Verfahren nach Anspruch 1, dadurch gekennzeichnet, daß man in einer ersten Stufe das wasserfreie organische- oder Mineralsalz und das Sequestrierungsmittel der Formel (I) in Abwesenheit irgendeines Lösungsmittels einsetzt und daß man in einer zweiten Stufe das aus der ersten Stufe resultierende Produkt und das genannte organische Lösungsmittel einsetzt.

6. Verfahren nach irgendeinem der vorstehenden Ansprüche, dadurch gekennzeichnet, daß in der Formel (I) $R_1$, $R_2$, $R_3$ und $R_4$ gleich oder unterschiedlich ein Wasserstoffatom oder einen Methylrest darstellen.

7. Verfahren nach irgendeinem der Ansprüche 1 bis 5, dadurch gekennzeichnet, daß in der Formel (I) n eine ganze Zahl größer oder gleich 0 und kleiner oder gleich 6 ist.

8. Verfahren nach irgendeinem der Ansprüche 1 bis 5, dadurch gekennzeichnet, daß in der Formel (I) $R_5$ einem Alkylrest mit 1 bis 4 Kohlenstoffatomen darstellt.

9. Verfahren nach irgendeinem der Ansprüche 1 bis 5, dadurch gekennzeichnet, daß in der Formel (I) $R_1$, $R_2$, $R_3$ und $R_4$ gleich oder unterschiedlich ein Wasserstoffatom oder einen Methylrest darstellen, n eine ganze Zahl größer oder gleich 0 und kleiner oder gleich 6 ist und $R_5$ einen Akylrest mit 1 bis 4 Kohlenstoffatomen darstellt.

10. Verfahren nach Anspruch 9, dadurch gekennzeichnet, daß das Sequestrierungsmittel der Formel (I) tris-(Dioxa-3,6-heptyl)amin der Formel:

$$N—(CH_2—CH_2—O—CH_2—CH_2—O—CH_3)_3$$

ist.

11. Verfahren nach Anspruch 9, dadurch gekennzeichnet, daß das Sequestrierungsmittel der Formel (I) das tris-(Trioxa-3,6,9-decyl)amin der Formel:

$$N—(CH_2—CH_2—O—CH_2—CH_2—O—CH_2—CH_2—O—CH_3)_3$$

ist.

12. Verfahren nach Anspruch 9, dadurch gekennzeichnet, daß das Sequestrierungsmittel der Formel (I) das tris-(Dioxa-3,6-octyl)amin der Formel:

$$N—(CH_2—CH_2—O—CH_2—CH_2—O—C_2H_5)_3$$

ist.

13. Verfahren nach Anspruch 9, dadurch gekennzeichnet, daß das Sequestrierungsmittel der Formel (I) das tris(Trioxa-3,6,9-undecyl)amin der Formel:

$$N—(CH_2—CH_2—O—CH_2—CH_2—O—CH_2—CH_2—O—C_2H_5)_3$$

ist.

14. Verfahren nach Anspruch 9, dadurch gekennzeichnet, daß das Sequestrierungsmittel der Formel (I) das tris(Dioxa-3,6-nonyl)amin der Formel:

$$N—(CH_2—CH_2—O—CH_2—CH_2—O—C_3H_7)_3$$

15. Verfahren nach Anspruch 9, dadurch gekennzeichnet, daß das Sequestrierungsmittel der ...mel (I) das tris(Trioxa-3,6,9-dodecyl) amin der Formel:

$$N—(CH_2—CH_2—O—CH_2—CH_2—O—CH_2—CH_2—O—C_3H_7)_3$$

...6. Verfahren nach Anspruch 9, dadurch gekennzeichnet, daß das Sequestrierungsmittel der ... (I) das tris(Dioxa-3,6-decyl) amin der Formel:

$$N-(CH_2-CH_2-O-CH_2-CH_2-O-C_4H_9)_3$$

ist.

17. Verfahren nach Anspruch 9, dadurch gekennzeichnet, daß das Sequestrierungsmittel der Formel (I) das tris(Trioxa-3,6,9-tridecyl)amin der Formel:

$$N-(CH_2-CH_2-O-CH_2-CH_2-O-CH_2-CH_2-O-C_4H_9)_3$$

ist.

18. Verfahren nach Anspruch 9, dadurch gekennzeichnet, daß das Sequestrierungsmittel der Formel (I) das tris(Oxa-3-butyl) amin der Formel:

$$N-(CH_2-CH_2-O-CH_3)_3$$

ist.

19. Verfahren nach Anspruch 9, dadurch gekennzeichnet, daß das Sequestrierungsmittel der Formel (I) das tris(Tetraoxa-3,6,9,12-tridecyl) amin der Formel:

$$N-(CH_2-CH_2-O-CH_2-CH_2-O-CH_2-CH_2-O-CH_2-CH_2-O-CH_3)_3$$

ist.

20. Verfahren nach Anspruch 9, dadurch gekennzeichnet, daß das Sequestrierungsmittel der Formel (I) das tris(Hexaoxa-3,6,9,12,15,18-nonadecyl) amin der Formel:

$$[N-[(CH_2-CH_2-O)_6-CH_3]_3]_V$$

ist.

21. Verfahren nach Anspruch 9, dadurch gekennzeichnet, daß das Sequestrierungsmittel der Formel (I) das tris(Dioxa-3,6-methyl-4-heptyl)amin der Formel:

$$N-(CH_2-CH_2-O-CHCH_3-CH_3-O-CH_3)_3$$

ist.

22. Verfahren nach Anspruch 9, dadurch gekennzeichnet, daß das Sequestrierungsmittel der Formel (I) das tris(Dioxa-3,6-dimethyl-2,4-heptyl) amin der Formel:

$$N-(CH_2-CHCH_3-O-CHCH_3-CH_2-O-CH_3)_3$$

ist.

23. Verfahren nach Anspruch 1, dadurch gekennzeichnet, daß das Kation $M^+$ aus der Gruppe ausgewählt ist, die das Kation $NH_4^+$, die Kationen $RNH_3^+$, wo R ein Alkylrest oder Arylrest ist und die Kationen der Metalle Li, Na, K, Rb, Cs, Mg, Ca, Sr, Ba, Sc, Y, La und die Lanthaniden, Ac und Actiniden, Ti, Zr, Hf, V, Nb, Ta, Cr, Mo, W, Mn, Tc, Re, Fe, Co, Ni, Ru, Rh, Pd, Os, Ir, Pt, Cu, Ag, Au, Sn, Cd, Hg, Al, Ga, In, Tl, Ge, Sn, Pb, Sb, Bi umfasst.

24. Verfahren nach Anspruch 1, dadurch gekennzeichnet, daß das Anion $A^-$ aus der Gruppe ausgewählt ist, die

$SCN^-$, $O{=}C{=}N^-$, $Cl^-$, $Br^-$, $I^-$, $F^-$, $CN^-$, $SH^-$, $S^=$, $OH^-$, $HSO_3^-$, $ClO_4^-$, $BrO_4^-$, $NH_2^-$,

$NO_3^-$, $NO_2^-$, $BF_4^-$, $BrO^-$, $ClO^-$, $BH_4^-$, $SO_3^{--}$, $PO_4^{3-}$, $CO_3^{--}$, $SO_4^{--}$, $ClO_3^-$, $BrO_3^-$, $H^-$, $AlH_4^-$.

umfasst.

25. Verfahren nach Anspruch 1, dadurch gekennzeichnet, daß das Anion $A^-$ aus der Gruppe ausgewählt ist, die die Anionen umfasst, die von Alkoholen, Phenolen, Thiolen, Thiophenolen, Säuren, Aminen, Amiden, organischen Verbindungen mit beweglichem Wasserstoff und Silanolen stammen.

26. Verfahren nach Anspruch 1, dadurch gekennzeichnet, daß das genannte organische Lösungsmittel aus der Gruppe ausgewählt ist, die Hexan, Cyclohexan, Benzol, Toluol, o-Xylol, m-Xylol, p-Xylol, Chlorbenzol, o-Dichlorbenzol, Trichloro-1,2,4-benzol, Chloroform, Methylenchlorid, Tetrachlorkohlenstoff, Dichloro - 1,2 - äthan, Trichloro - 1,1,1 - äthan, Trichloro - 1,1,2 - äthan, Trichloro - 1,2,2-trifluoro - 1,1,2 - äthan, Perchloräthylen, Acetonitril, Dimethylformamid, N-Methylpyrrolidon, Dimethylacetamid, Hexamethylphosphorotriamid, Dimethylsulfoxid, Sulfolan, Methanol, Äthanol und Isopropanol umfasst.

27. Verfahren nach Anspruch 1, dadurch gekennzeichnet, daß man bei einer Temperatur zwischen −50°C und 250°C arbeitet.

28. Verfahren nach Anspruch 1, dadurch gekennzeichnet, daß man das Sequestrierungsmittel der

Formel (I) in einer solchen Menge verwendet, daß das Molverhältnis des Sequestrierungsmittels zum Salz $A^-M^+$ zwischen 0,001 und 50 liegt.

29. Anwendung des Verfahrens nach irgendeinem der vorstehenden Ansprüche zum Einbringen des Salzes $A^-M^+$ in Lösung in dem genannten organischen Lösungsmittel in eine organische Synthesereaktion.

30. Anwendung des Verfahrens nach irgendeinem der Ansprüche 1 bis 28 zur Extraktion des Salzes $A^-M^+$ aus einer dieses enthaltenden Lösung.

31. Komplex der Formel:

$$[N[CHR_1—CHR_2—O—(CHR_3—CHR_4—O)_nR_5]_3]_y\ (M^+A^-)\ (II)$$

in dem y größer oder gleich 1 und kleiner oder gleich 3 ($1 \leqslant y \leqslant 3$) ist und in der n eine ganze Zahl größer oder gleich 0 und kleiner oder gleich 10 ist ($0 \leqslant n \leqslant 10$), $R_1$, $R_2$, und $R_4$ gleich oder unterschiedlich ein Wasserstoffatom oder einen Alkylrest mit 1 bis 4 Kohlenstoffatomen darstellen und $R_5$ einen Alkylrest oder Cycloalkylrest mit 1 bis 12 Kohlenstoffatomen, einen Phenylrest oder einen Rest $—C_m H_{2m}—\phi$ oder $C_m— H_{2m+1}—\phi—$ ($1 \leqslant m \leqslant 12$), darstellt, A ein Mineral- oder organisches Anion und $M^+$ ein Kation darstellt, das aus der Gruppe ausgewählt ist, die das Kation $NH_4^+$ und seine Derivate und die Kationen umfasst, die von den Metallen der Gruppen $I_A$, $II_A$, $III_A$, $IV_A$, $V_A$, $VI_A$, $VII_A$, VIII, $I_B$, $II_B$, $III_B$, $IV_B$ und $V_B$ des Periodensystems der Elemente abstammen.

32. Komplex nach Anspruch 31, dadurch gekennzeichnet, daß in der Formel (II) $R_1$, $R_2$, $R_3$ und $R_4$ gleich oder unterschiedlich ein Wasserstoffatom oder einen Methylrest darstellen.

33. Komplex nach Anspruch 31, dadurch gekennzeichnet, daß in der Formel (II) n eine ganze Zahl größer oder gleich 0 und kleiner oder gleich 6 ist.

34. Komplex nach Anspruch 31, dadurch gekennzeichnet, daß in der Formel (II) $R_5$ einen Alkylrest mit 1 bis 4 Kohlenstoffatomen darstellt.

35. Komplex nach Anspruch 31, dadurch gekennzeichnet, daß in der Formel (II) $R_1$, $R_2$, $R_3$ und $R_4$ gleich oder unterschiedlich ein Wasserstoffatom oder einen Methylrest darstellen, n eine ganze Zahl größer oder gleich 0 und kleiner oder gleich 6 ist und $R_5$ einen Alkylrest mit 1 bis 4 Kohlenstoffatomen darstellt.

36. Komplex nach Anspruch 33, dadurch gekennzeichnet, daß er der Formel:

$$[N—(CH_2—CH_2—O—CH_2—CH_2—O—CH_3)_3]_y\ (A^-M^+)$$

entspricht.

37. Komplex nach Anspruch 35, dadurch gekennzeichnet, daß er der Formel:

$$[N—(CH_2—CH_2—O—CH_2—CH_2—O—CH_2—CH_2—O—CH_3)_3]_y\ (A^-M^+)$$

entspricht.

38. Komplex nach Anspruch 35, dadurch gekennzeichnet, daß er der Formel:

$$[N—(CH_2—CH_2—O—CH_2—CH_2—O—C_2H_5)_3]_y\ (A^-M^+)$$

entspricht.

39. Komplex nach Anspruch 35, dadurch gekennzeichnet, daß er der Formel:

$$[N—(CH_2—CH_2—O—CH_2—CH_2—O—CH_2—CH_2—O—C_2H_5)_3]_y\ (A^-M^+)$$

entspricht.

40. Komplex nach Anspruch 35, dadurch gekennzeichnet, daß er der Formel:

$$[N—(CH_2—CH_2—O—CH_2—CH_2—O—C_3H_7)_3]_y\ (A^-M^+)$$

entspricht.

41. Komplex nach Anspruch 35, dadurch gekennzeichnet, daß er der Formel:

$$[N—(CH_2—CH_2—O—CH_2—CH_2—O—CH_2—CH_2—O—C_3H_7)_3]_y\ (A^-M^+)$$

entspricht.

42. Komplex nach Anspruch 35, dadurch gekennzeichnet, daß er der Formel:

$$[N—(CH_2—CH_2—O—CH_2—CH_2—O—C_4H_9)_3]_y\ (A^-M^+)$$

entspricht.

43. Komplex nach Anspruch 35, dadurch gekennzeichnet, daß er der Formel:

$$[N-(CH_2-CH_2-O-CH_2-CH_2-O-CH_2-CH_2-O-C_4H_9)_3]_y\ (A^-M^+)$$

entspricht.

44. Komplex nach Anspruch 35, dadurch gekennzeichnet, daß er der Formel:

$$[N-(CH_2-CH_2-O-CH_3)_3]_y\ (A^-M^+)$$

entspricht.

45. Komplex nach Anspruch 35, dadurch gekennzeichnet, daß er der Formel:

$$[N-(CH_2-CH_2-O-CH_2-CH_2-O-CH_2-CH_2-O-CH_2-CH_2-O-CH_3)_3]_y\ (A^-M^+)$$

entspricht.

46. Komplex nach Anspruch 35, dadurch gekennzeichnet, daß er der Formel:

$$[N-[(CH_2-CH_2-O)_6-CH_3]_3]_y\ (A^-M^+)$$

entspricht.

47. Komplex nach Anspruch 35, dadurch gekennzeichnet, daß er der Formel:

$$[N-(CH_2-CH_2-O-CH_2-CH_2-O-CH_2-CH_2-O-C_4H_9)_3]_y\ (A^-M^+)$$

entspricht.

48. Komplex nach Anspruch 35, dadurch gekennzeichnet, daß er der Formel:

$$[N-(CH_2-CH_2-O-CH_3)_3]_y\ (A^-M^+)$$

entspricht.

49. Komplex nach irgendeinem der Ansprüche 31 bis 48, dadurch gekennzeichnet, daß in der Formel (II) das Kation $M^+$ aus der Gruppe ausgewählt ist, die das Kation $NH_4^+$, die Kationen $RNH_3^+$, wo R ein Alkylrest oder Arylrest ist und die Kationen umfaßt, die von den Metallen Li, Na, K, Rb, Cs, Mg, Ca, Sr, Ba, Sc, Y, La und Lanthaniden, Ac und Actiniden, Ti, Zr, Hf, V, Nb, Ta, Cr, Mo, W, Mn, Tc, Re, Fe, Co, Ni, Ru, Rh, Pd, Os, Ir, Pt, Cu, Ag, Au, Zn, Cd, Hg, Al, Ga, In, Tl, Ge, Sn, Pb, Sb, Bi abgeleitet sind.

50. Komplex nach irgendeinem der Ansprüche 31 bis 49, dadurch gekennzeichnet, daß in der Formel (II) das Anion $A^-$ aus der Gruppe ausgewählt ist, die

$$SCN^-,\ O{=}C{=}N^-,\ Cl^-,\ Br^-,\ I^-,\ F^-,\ CN^-,\ SH^-,\ S^{=},\ OH^-,\ HSO_3^-,\ ClO_4^-,\ BrO_4^-,\ NH_2^-,$$

$$NO_3^-,\ NO_2^-,\ BF_4^-,\ H^-,\ BrO^-,\ ClO^-,\ BH_4^-,\ SO_3^{--},\ PO_4^{3-};\ CO_3^{--},\ SO_4^{--},\ ClO_3^-,\ BrO_3^-,\ AlH_4^-.$$

51. Komplex nach irgendeinem der Ansprüche 31 bis 49, dadurch gekennzeichnet, daß in der Formel (II) das Anion $A^-$ aus der Gruppe ausgewählt ist, die die Anionen umfaßt, die von Alkoholen, Phenolen, Thiolen, Thiophenolen, Säuren, Aminen, Amiden, organischen Verbindungen mit beweglichem Wasserstoff und Silanolen abstammen.

**Claims for the Contracting States: BE, CH, DE, FR, GB, IT, LU, NL, SE**

1. Process for solubilising an organic or inorganic salt in an organic solvent in which the organic or inorganic salt is initially insoluble, or for increasing the solubility of an organic or inorganic salt in an organic solvent, characterised in that the organic or inorganic salt of the formula $A^-M^+$, in which $A^-$ represents an inorganic or organic anion and $M^+$ represents a cation chosen from amongst the group comprising the cation $NH_4^+$ and its derivatives and the cations derived from the metals of groups $I_A$, $II_A$, $III_A$, $IV_A$, $V_A$, $VI_A$, $VII_A$, VIII, $I_B$, $II_B$, $III_B$, $IV_B$ and $V_B$ of the periodic classification of the elements, is brought together with at least one sequestering agent, which is soluble in the said organic solvent of the formula: $N-[CHR_1-CHR_2-O-(CHR_3-CHR_4-O)_n-R_5]_3$ (I), in which n is an integer which is greater than or equal to 0 and less than or equal to 10 ($0 \leqslant n \leqslant 10$), $R_1$, $R_2$, $R_3$ and $R_4$, which are identical or different, represent a hydrogen atom or an alkyl radical having from 1 to 4 carbon atoms and $R_5$ represents an alkyl or cycloalkyl radical having from 1 to 12 carbon atoms, a phenyl radical or a radical $-C_mH_{2m}-\phi$ or $C_mH_{2m+1}-\phi-$, in which m is between 1 and 12, the sequestering agent of the formula (I) and the organic or inorganic salt forming a complex of the formula: $[N-[CHR_1-CHR_2-O-(CHR_3-CHR_4-O-)_nR_5]_3]_y\ (M^+A^-)$ (II), in which y is greater than or equal to 1 and less than or equal to 3 ($1 \leqslant y \leqslant 3$), and this complex of the formula (II) being soluble in the said organic solvent.

2. A process according to Claim 1, characterised in that the anhydrous organic or inorganic salt is brought together in a single step with a solution of the sequestering agent of the formula (I) in the said organic solvent.

3. A process according to Claim 1, characterized in that an aqueous solution of the organic or inorganic salt is brought together in a single step with a solution of the sequestering agent of the formula (I) in the said organic solvent.

4. Process according to Claim 1, characterised in that, in a first step, a solution of the inorganic or organic salt in an auxiliary solvent is brought together with a solution of the sequestering agent of the formula (I) in the same auxiliary solvent, in that, in a second step, the auxiliary solvent is removed, and in that, in a third step, the product resulting from the second step is brought together with the said solvent.

5. A process according to Claim 1 characterised in that, in a first step, the anhydrous inorganic or organic salt is brought together with the sequestering agent of the formula (I) in the absence of any solvent, and in that, in a second step, the product resulting from the first step is brought together with the said organic solvent.

6. Process according to any one of the preceding claims, characterised in that, in the formula (I), $R_1$, $R_2$, $R_3$ and $R_4$, which are identical or different, represent a hydrogen atom or a methyl radical.

7. Process according to any one of Claims 1 to 5, characterised in that, in the formula (I), n is an integer which is greater than or equal to 0 and less than or equal to 6.

8. Process according to any one of Claims 1 to 5, characterised in that, in the formula (I), $R_5$ represents an alkyl radical having from 1 to 4 carbon atoms.

9. Process according to any one of Claims 1 to 5, characterised in that, in the formula (I), $R_1$, $R_2$, $R_3$ and $R_4$, which are identical or different, represent a hydrogen atom or a methyl radical, n is an integer which is greater than or equal to 0 and less than or equal to 6 and $R_5$ represents an alkyl radical having from 1 to 4 carbon atoms.

10. Process according to Claim 9, characterised in that the sequestering agent of the formula (I) is tris-(3,6-dioxaheptyl)-amine of the formula:

$$N—(CH_2—CH_2—O—CH_2—CH_2—O—CH_3)_3.$$

11. Process according to Claim 9, characterised in that the sequestering agent of the formula (I) is tris-(3,6,9-trioxadecyl)-amine of the formula:

$$N—(CH_2—CH_2—O—CH_2—CH_2—O—CH_2—CH_2—O—CH_3)_3.$$

12. Process according to Claim 9, characterised in that the sequestering agent of the formula (I) is tris(3,6-dioxaoctyl)-amine of the formula:

$$N—(CH_2—CH_2—O—CH_2—CH_2—O—C_2H_5)_3.$$

13. Process according to Claim 9, characterised in that the sequestering agent of the formula (I) is tris-(3,6,9-trioxaundecyl)-amine of the formula:

$$N—(CH_2—CH_2—O—CH_2—CH_2—O—CH_2—CH_2—O—C_2H_5)_3.$$

14. Process according to Claim 9, characterised in that the sequestering agent of the formula (I) is tris-(3,6-dioxanonyl)-amine of the formula:

$$N—(CH_2—CH_2—O—CH_2—CH_2—O—C_3H_7)_3.$$

15. Process according to Claim 9, characterised in that the sequestering agent of the formula (I) is tris-(3,6,9-trioxadodecyl)-amine of the formula:

$$N—(CH_2—CH_2—O—CH_2—CH_2—O—CH_2—CH_2—O—C_3H_7)_3.$$

16. Process according to Claim 9, characterised in that the sequestering agent of the formula (I) is tris-(3,6-dioxadecyl)-amine of the formula:

$$N—(CH_2—CH_2—O—CH_2—CH_2—O—C_4H_9)_3.$$

17. Process according to Claim 9, characterised in that the sequestering agent of the formula (I) is tris-(3,6,9-trioxatridecyl)-amine of the formula:

$$N—(CH_2—CH_2—O—CH_2—CH_2—O—CH_2—CH_2—O—C_4H_9)_3.$$

18. Process according to Claim 9 characterised in that the sequestering agent of the formula (I) is tris-(3-oxabutyl)-amine of the formula:

$$N-(CH_2-CH_2-O-CH_3)_3.$$

19. Process according to Claim 9, characterised in that the sequestering agent of the formula (I) is tris-(3,6,9,12-tetraoxatridecyl)-amine of the formula:

$$N-(CH_2-CH_2-O-CH_2-CH_2-O-CH_2-CH_2-O-CH_2-CH_2-O-CH_3)_3.$$

20. Process according to Claim 9, characterised in that the sequestering agent of the formula (I) is tris - (3,6,9,12,15,18 - hexaoxanonadecyl) - amine of the formula:

$$[N-[(CH_2-CH_2-O)_6-CH_3]_3]_y.$$

21. Process according to Claim 9, characterised in that the sequestering agent of the formula (I) is tris - (3,6 - dioxa - 4 - methylheptyl) - amine of the formula:

$$N-(CH_2-CH_2-O-CHCH_3-CH_2-O-CH_3)_3.$$

22. Process according to Claim 9, characterised in that the sequestering agent of the formula (I) is tris - (3,6 - dioxa - 2,4 - dimethylheptyl) - amine of the formula:

$$N-(CH_2-CHCH_3-O-CHCH_3-CH_2-O-CH_3)_3.$$

23. Process according to Claim 1, characterised in that the cation $M^+$ is chosen from amongst the group comprising the cation $NH_4^+$, the cations $RNH_3^+$, in which R is an alkyl or aryl radical, and the cations derived from the metals Li, Na, K, Rb, Cs, Mg, Ca, Sr, Ba, Sc, Y, La and lanthanides, Ac and actinides, Ti, Zr, Hf, V, Nb, Ta, Cr, Mo, W, Mn, Tc, Re, Fe, Co, Ni, Ru, Rh, Pd, Os, Ir, Pt, Cu, Ag, Au, Zn, Cd, Hg, Al, Ga, In, Tl, Ge, Sn, Pb, Sb and Bi.

24. Process according to Claim 1, characterised in that the anion $A^-$ is chosen from amongst the group comprising

$$SCN^-,\ O{=}C{=}N^-,\ Cl^-,\ Br^-,\ I^-,\ F^-,\ CN^-,\ SH^-,\ S^{--},\ OH^-,\ HSO_3^-,\ ClO_4^-,\ BrO_4^-,\ NH_2^-,$$

$$NO_3^-,\ NO_2^-,\ BF_4^-,\ BrO^-,\ ClO^-,\ BH_4^-,\ SO_3^{--},\ PO_4^{3-},CO_3^{--},\ SO_4^{--},\ ClO_3^-,\ BrO_3^-,\ H^-,\ AlH_4^-.$$

25. Process according to Claim 1, characterised in that the anion $A^-$ is chosen from amongst the group comprising the anions derived from alcohols, phenols, thiols thiophenols, acids, amines, amides, organic compounds with a mobile hydrogen, and silanols.

26. Process according to Claim 1, characterised in that the said organic solvent is chosen from amongst the group comprising hexane, cyclohexane, benzene, toluene, o-xylene, m-xylene, p-xylene, chlorobenzene, o-dichlorobenzene, 1,2,4-trichlorobenzene, chloroform, methylene chloride, carbon tetrachloride, 1,2-dichloroethane 1,1,1-trichloroethane, 1,1,2-trichloroethane, 1,2,2 - trichloro - 1,1,2 - trifluoroethane, perchloroethylene, acetonitrile, dimethylformamide, N-methylpyrrolidone, dimethylacetamide, hexamethylphosphorotriamide, dimethylsulphoxide, sulpholane, methanol, ethanol and isopropanol.

27. Process according to Claim 1, characterised in that the reaction is carried out at a temperature between −50°C and 250°C.

28. Process according to Claim 1, characterised in that the sequestering agent of the formula (I) is used in amounts such that the molar ratio of the sequestering agent to the salt $A^-M^+$ is between 0.001 and 50.

29. Application of the process according to any one of the preceding claims to the use of a solution of the salt $A^-M^+$ in the said organic solvent, in an organic synthesis reaction.

30. Application of the process according to any one of Claims 1 to 28 to the extraction of the salt $A^-M^+$ from a solution in which it is present.

31. Complex of the formula: $[N[CHR_1-CHR_2-O-(CHR_3-CHR_4-O)_nR_5]_3]_y\ (M^+A^-)$ (II) in which y is greater than or equal to 1 and less than or equal to 3 ($1 \leqslant y \leqslant 3$) and in which n is an integer which is greater than or equal to 0 and less than or equal to 10 ($0 \leqslant n \leqslant 10$), $R_1$, $R_2$, $R_3$ and $R_4$, which are identical or different, represent a hydrogen atom or an alkyl radical having from 1 to 4 carbon atoms and $R_5$ represents an alkyl or cycloalkyl radical having from 1 to 12 carbon atoms, a phenyl radical or a radical $-C_mH_{2m}-\phi$ or $C_m-H_{2m+1}-\phi-$ ($1 \leqslant m \leqslant 12$), $A^-$ representing an inorganic or organic anion and $M^+$ representing a cation chosen from amongst the group comprising the cation $NH^+$ and its derivatives and the cations derived from the metals of groups $I_A$, $II_A$, $III_A$, $IV_A$, $V_A$, $VI_A$, $VII_A$, VIII, $I_B$, $II_B$, $III_B$, $IV_B$ and $V_B$ of the periodic classification of the elements.

32. Complex according to Claim 31, characterised in that, in the formula II, $R_1$, $R_2$, $R_3$, and $R_4$, which are identical or different, represent a hydrogen atom or a methyl radical.

33. Complex according to Claim 31, characterised in that, in the formula II, n is an integer which is

greater than or equal to 0 and less than or equal to 6.

34. Complex according to Claim 31, characterised in that, in the formula II, $R_5$ represents an alkyl radical having from 1 to 4 carbon atoms.

35. Complex according to Claim 31, characterised in that, in the formula II, $R_1$, $R_2$, $R_3$ and $R_4$, which are identical or different, represent a hydrogen atom or a methyl radical, n is an integer which is greater than or equal to 0 and less than or equal to 6 and $R_5$ represents an alkyl radical having from 1 to 4 carbon atoms.

36. Complex according to Claim 33, characterised in that it corresponds to the formula:

$$[N-(CH_2-CH_2-O-CH_2-CH_2-O-CH_3)_3]_y\ (A^-M^+).$$

37. Complex according to Claim 35, characterised in that it corresponds to the formula:

$$[N-(CH_2-CH_2-O-CH_2-CH_2-O-CH_2-CH_2-O-CH_3)_3]_y\ (A^-M^+)$$

38. Complex according to Claim 35, characterised in that it corresponds to the formula:

$$[N-(CH_2-CH_2-O-CH_2-CH_2-O-C_2H_5)_3]_y\ (A^-M^+)$$

39. Complex according to Claim 35, characterised in that it corresponds to the formula:

$$[N-(CH_2-CH_2-O-CH_2-CH_2-O-CH_2-CH_2-O-C_2H_5)_3]_y\ (A^-M^+).$$

40. Complex according to Claim 35, characterised in that it corresponds to the formula:

$$[N-(CH_2-CH_2-O-CH_2-CH_2-O-C_3H_7)_3]_y\ (A^-M^+).$$

41. Complex according to Claim 35, characterised in that it corresponds to the formula:

$$[N-(CH_2-CH_2-O-CH_2-CH_2-O-CH_2-CH_2-O-C_3H_7)_3]_y\ (A^-M^+).$$

42. Complex according to Claim 35, characterised in that it corresponds to the formula:

$$[N-(CH_2-CH_2-O-CH_2-CH_2-O-C_4H_9)_3]_y\ (A^-M^+).$$

43. Complex according to Claim 35, characterised in that it corresponds to the formula:

$$[N-(CH_2-CH_2-O-CH_2-CH_2-O-CH_2-CH_2-O-C_4H_9)_3]_y\ (A^-M^+).$$

44. Complex according to Claim 35, characterised in that it corresponds to the formula:

$$[N-(CH_2-CH_2-O-CH_3)_3]_y\ (A^-M^+).$$

45. Complex according to Claim 35, characterised in that it corresponds to the formula:

$$[N-(CH_2-CH_2-O-CH_2-CH_2-O-CH_2-CH_2-O-CH_2-CH_2-O-CH_3)_3]_y\ (A^-M^+).$$

46. Complex according to Claim 35, characterised in that it corresponds to the formula:

$$[N-[(CH_2-CH_2-O)_6-CH_3]_3]_y\ (A^-M^+).$$

47. Complex according to Claim 35, characterised in that it corresponds to the formula:

$$[N-(CH_2-CH_2-O-CH_2-CH_2-O-CH_2-CH_2-O-C_4H_9)_3]_y\ (A^-M^+).$$

48. Complex according to Claim 35, characterised in that it corresponds to the formula:

$$[N-(CH_2-CH_2-O-CH_3)_3]_y\ (A^-M^+).$$

49. Complex according to any one of Claims 31 to 48 characterised in that, in the formula II, the cation $M^+$ is chosen from amongst the group comprising the cation $NH_4^+$, the cations $RNH_3^+$, in which R is an alkyl or aryl radical, and the cations derived from the metals Li, Na, K, Rb, Cs, Mg, Ca, Sr, Ba, Sc, Y, La and lanthanides, Ac and actinides, Ti, Zr, Hf, V, Nb, Ta, Cr, Mo, W, Mn, Tc, Re, Fe, Co, Ni, Ru, Rh, Pd, Os, Ir, Pt, Cu, Ag, Au, Zn, Cd, Hg, Al, Ga, In, Tl, Ge, Sn, Pb, Sb and Bi.

50. Complex according to any one of Claims 31 to 49, characterised in that, in the formula II, the

anion $A^-$ is chosen from amongst the group comprising

$SCN^-$, $O{=}C{=}N^-$, $Cl^-$, $Br^-$, $I^-$, $F^-$, $CN^-$, $SH^-$, $S^{--}$, $OH^-$, $HSO_3^-$, $ClO_4^-$, $BrO_4^-$, $NH_2^-$,

$NO_3^-$, $NO_2^-$, $BF_4^-$, $H^-$, $BrO^-$, $ClO^-$, $BH_4^-$, $SO_3^{--}$, $PO_4^{3-}$, $CO_3^{--}$, $SO_4^{--}$, $ClO_3^-$, $BrO_3^-$, $AlH_4^-$.

51. Complex according to any one of Claims 31 to 49, characterised in that, in the formula II, the anion $A^-$ is chosen from amongst the group comprising the anions derived from alcohols, phenols, thiols, thiophenols, acids, amines, amides, organic compounds with a mobile hydrogen, and silanols.

**Revendications pour l'Etat contractant: AT**

1. Procédé pour solubiliser un sel organique ou minéral dans un solvant organique dans lequel le sel organique ou minéral n'est initialement pas soluble ou pour augmenter la solubilité d'un sel organique ou minéral dans un solvant organique, caractérisé en ce que l'on met en présence le sel organique ou minéral de formule $A^-M^+$ dans laquelle $A^-$ représente un anion minéral ou organique et $M^+$ représente un cation choisi parmi le groupe comprenant le cation $NH_4^+$ et ses dérivés et les cations dérivés des métaux des groupes $I_A$, $II_A$, $III_A$, $IV_A$, $V_A$, $VI_A$, $VII_A$, VIII, $I_B$, $II_B$, $III_B$, $IV_B$ et $V_B$ de la classification périodique des éléments et au moins un agent séquestrant, soluble dans ledit solvant organique, de formule:

$$N—[CHR_1—CHR_2—O—(CHR_3—CHR_4—O)_n—R_5]_3 \qquad (I)$$

dans laquelle n est un nombre entier supérieur ou égal à 0 et inférieur ou égal à 10 ($0 \leqslant n \leqslant 10$), $R_1$ $R_2$ $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $—C_m H_{2m}—\emptyset$ ou $C_mH_{2m+1}—\emptyset—$, où m est compris entre 1 et 12, l'agent séquestrant de formule (I) et le sel organique ou minéral formant un complexe de formule:

$$[N—[CHR_1—CHR_2—O—(CHR_3—CHR_4—O—)_nR_5]_3]_y(M^+ A^-) \qquad (II)$$

dans laquelle y est supérieur ou égal à 1 et inférieur ou égal à 3 ($1 \leqslant y \leqslant 3$), ce complexe de formule (II) étant soluble dans ledit solvant organique.

2. Procédé selon la revendication 1 caractérisé en ce que l'on met en présence en une seule étape le sel organique ou minéral anhydre et l'agent séquestrant de formule (I) en solution dans ledit solvant organique.

3. Procédé selon la revendication 1 caractérisé en ce que l'on met en présence en une seule étape le sel organique ou minéral en solution aqueuse et l'agent séquestrant de formule (I) en solution dans ledit solvant organique.

4. Procédé selon la revendication 1 caractérisé en ce que l'on met en présence, dans une première étape, le sel minéral ou organique en solution dans un tiers solvant et l'agent séquestrant de formule (I) en solution dans le même tiers solvant, en ce que l'on élimine, dans une deuxième étape, le tiers solvant et en ce que l'on met en présence, dans une troisième étape, le produit résultant de la deuxième étape et ledit solvant.

5. Procédé selon la revendication 1, caractérisé en ce que l'on met en présence, dans une première étape, le sel minéral ou organique anhydre et l'agent séquestrant de formule (I) en l'absence de tout solvant et en ce que l'on met en présence, dans une deuxième étape, le produit résultant de la première étape et ledit solvant organique.

6. Procédé selon l'une quelconque des revendications précédentes caractérisé en ce que dans la formule (I), $R_1$ $R_2$ $R_3$ et $R_4$ identiques ou différents représentent un atome d'hydrogène ou un radical méthyle.

7. Procédé selon l'une quelconque des revendications 1 à 5 caractérisé en ce que dans la formule (I) n est un nombre entier supérieur ou égal à 0 et inférieur ou égal à 6.

8. Procédé selon l'une quelconque des revendications 1 à 5 caractérisé en ce que dans la formule (I) $R_5$ représente un radical alkyle ayant de 1 à 4 atomes de carbone.

9. Procédé selon l'une quelconque des revendications 1 à 5 caractérisé en ce que dans la formule (I) $R_1$ $R_2$ $R_3$ et $R_4$ identiques ou différents représentent un atome d'hydrogène ou un radical méthyle, n est un nombre entier supérieur ou égal à 0 et inférieur ou égal à 6 et $R_5$ représente un radical alkyle ayant de 1 à 4 atomes de carbone.

10. Procédé selon la revendication 9 caractérisé en ce que l'agent séquestrant de formule (I) est la tris(dioxa-3,6 heptyl)amine de formule:

$$N—(CH_2—CH_2—O—CH_2—CH_2—O—CH_3)_3$$

11. Procédé selon la revendication 9 caractérisé en ce que l'agent séquestrant de formule (I) est la tris(trioxa-3,6,9 décyl)amine de formule:

$$N—(CH_2—CH_2—O—CH_2—CH_2—O—CH_2—CH_2—O—CH_3)_3$$

12. Procédé selon la revendication 9 caractérisé en ce que l'agent séquestrant de formule (I) est la tris(dioxa-3,6 octyl)amine de formule:

$$N—(CH_2—CH_2—O—CH_2—CH_2—O—C_2H_5)_3$$

13. Procédé selon la revendication 9 caractérisé en ce que l'agent séquestrant de formule (I) est la tris(trioxa-3,6,9 undécyl)amine de formule:

$$N-(CH_2-CH_2-O-CH_2-CH_2-O-CH_2-CH_2-O-C_2H_5)_3$$

14. Procédé selon la revendication 9 caractérisé en ce que l'agent séquestrant de formule (I) est la tris(dioxa-3,6 nonyl)amine de formule:

$$N-(CH_2-CH_2-O-CH_2-CH_2-O-C_3H_7)_3$$

15. Procédé selon la revendication 9 caractérisé en ce que l'agent séquestrant de formule (I) est la tris(trioxa-3,6,9 dodécyl)amine de formule:

$$N-(CH_2-CH_2-O-CH_2-CH_2-O-CH_2-CH_2-O-C_3H_7)_3$$

16. Procédé selon la revendication 9 caractérisé en ce que l'agent séquestrant de formule (I) est la tris(dioxa-3,6 décyl)amine de formule:

$$N-(CH_2-CH_2-O-CH_2-CH_2-O-C_4H_9)_3$$

17. Procédé selon la revendication 9 caractérisé en ce que l'agent séquestrant de formule (I) est la tris(trioxa-3,6,9 tridécyl)amine de formule:

$$N-(CH_2-CH_2-O-CH_2-CH_2-O-CH_2-CH_2-O-C_4H_9)_3$$

18. Procédé selon la revendication 9 caractérisé en ce que l'agent séquestrant de formule (I) est la tris(oxa-3 butyl)amine de formule:

$$N-(CH_2-CH_2-O-CH_3)_3$$

19. Procédé selon la revendication 9 caractérisé en ce que l'agent séquestrant de formule (I) est la tris(tétraoxa-3,6,9,12 tridécyl)amine de formule:

$$N-(CH_2-CH_2-O-CH_2-CH_2-O-CH_2-CH_2-O-CH_2-CH_2-O-CH_3)_3$$

20. Procédé selon la revendication 9 caractérisé en ce que l'agent séquestrant de formule (I) est la tris(hexaoxa-3,6,9,12,15,18 nonadécyl)amine de formule:

$$[N-[(CH_2-CH_2-O)_6-CH_3]_3]_Y$$

21. Procédé selon la revendication 9 caractérisé en ce que l'agent séquestrant de formule (I) est la tris(dioxa-3,6 méthyl-4 heptyl)amine de formule:

$$N-(CH_2-CH_2-O-CHCH_3-CH_2-O-CH_3)_3$$

22. Procédé selon la revendication 9 caractérisé en ce que l'agent séquestrant de formule (I) est la tris(dioxa-3,6 diméthyl-2,4 heptyl)amine de formule:

$$N-(CH_2-CHCH_3-O-CHCH_3-CH_2-O-CH_3)_3$$

23. Procédé selon la revendication 1 caractérisé en ce que le cation $M^+$ est choisi parmi le groupe comprenant le cation $NH_4^+$, les cations $RNH_3^+$ où R est un radical alkyle ou aryle et les cations dérivés des métaux Li, Na, K, Rb, Cs, Mg, Ca, Sr, Ba, Sc, Y, La et lanthanides, Ac et actinides, Ti, Zr, Hf, V, Nb, Ta, Cr, Mo, W, Mn, Tc, Re, Fe, Co, Ni, Ru, Rh, Pd, Os, Ir, Pt, Cu, Ag, Au, Zn, Cd, Hg, Al, Ga, In, Tl, Ge, Sn, Pb, Sb, Bi.

24. Procédé selon la revendication 1 caractérisé en ce que l'anion $A^-$ est choisi parmi le groupe comprenant,

$$SCN^-,\ O{=}C{=}N^-,\ Cl^-,\ Br^-,\ I^-,\ F^-,\ CN^-,\ SH^-,\ S^{=},\ OH^-,\ HSO_3^-,\ ClO_4^-,\ BrO_4^-,\ NH_2^-,$$

$$NO_3^-,\ NO_2^-,\ BF_4^-,\ BrO^-,\ ClO^-,\ BH_4^-,\ SO_3^{--},\ PO_4^{3-},\ CO_3^{--},\ SO_4^{--},\ ClO_3^-,\ BrO_3^-,\ H^-,\ AlH_4^-.$$

25. Procédé selon la revendication 1 caractérisé en ce que l'anion $A^-$ est choisi parmi le groupe comprenant les anions dérivés des alcools, des phénols, des thiols, des thiophénols, des acides, des amines, des amides, des composés organiques à hydrogène mobile et des silanols.

26. Procédé selon la revendication 1 caractérisé en ce que ledit solvant organique est choisi parmi le groupe comprenant l'hexane, le cyclohexane, le benzène, le toluène, l'o-xylène, le m-xylène, le p-xylène, le chlorobenzène, l'o-dichlorobenzène, le trichloro-1,2,4-benzène, le chloroforme, le chlorure de méthylène, le tétrachlorure de carbone, le dichloro-1;2 éthane, le trichloro-1,1,1 éthane, le trichloro-1,1,2 éthane, le trichloro-1,2,2 trifluoro-1,1,2 éthane, le perchloroéthylène, l'acétonitrile, la diméthylformamide, le N-méthylpyrrolidone, la diméthylacétamide, l'hexaméthylphosphorotriamide, le diméthylsulfoxyde, le sulfolane, le méthanol, l'éthanol et l'isopropanol.

27. Procédé selon la revendication 1 caractérisé en ce qu'on opère à une température comprise entre −50°C et 250°C.

28. Procédé selon la revendication 1 caractérisé en ce qu'on utilise l'agent séquestrant de formule (I) en quantité telle que le rapport molaire de l'agent séquestrant au sel $A^- M^+$ est compris entre 0,001 et 50.

29. Application du procédé selon l'une quelconque des revendications précédentes à la mise oeuvre du sel $A^- M^+$ en solution dans ledit solvant organique, dans une réaction de synthèse organique.

30. Application du procédé selon l'une quelconque des revendications 1 à 28 à l'extraction du sel $A^- M^+$ d'une solution le contenant.

**Patentansprüche für den Vertragsstaat: AT**

1. Verfahren zum Lösen eines organischen- oder Mineralsalzes in einem organischen Lösungsmittel, in dem das organische- oder Mineralsalz nicht unmittelbar löslich ist oder um die Löslichkeit eines organischen- oder Mineralsalzes in einem organischen Lösungsmittel zu steigern, dadurch gekennzeichnet, daß man das organische- oder Mineralsalz der Formel $A^-M^+$, in der $A^-$ ein mineralisches oder organisches Anion und $M^+$ ein Kation darstellt, das aus der Gruppe ausgewählt ist, die das Kation $NH_4^+$ und seine Derivate sowie die Kationen der Metalle der Gruppen $I_A$, $II_A$, $III_A$, $IV_A$, $V_A$, $VI_A$, $VII_A$, VIII, $I_B$, $II_B$, $III_B$, $IV_B$ und $V_B$ des Periodensystems umfasst und mindestens ein Sequestrierungsmittel einsetzt, das in dem genannten organischen Lösungsmittel löslich ist, mit der Formel $N—[CHR_1—CHR_2—O—(CHR_3—CHR_4—O)_n—R_5]_3$ (I), in der n eine ganze Zahl größer oder gleich 0 und kleiner oder gleich 10 ist ($0 \leqslant n \leqslant 10$), $R_1$, $R_2$, $R_3$ und $R_4$ gleich oder unterschiedlich ein Wasserstoffatom oder einen Alkylrest mit 1 bis 4 Kohlenstoffatomen darstellen und $R_5$ ein Alkylrest oder Cycloalkylrest mit 1 bis 12 Kohlenstoffatomen, ein Phenylrest oder ein Radikal $—C_m H_{2m}—\emptyset$ oder $C_mH_{2m+1}—\emptyset—$ ist, wo m zwischen 1 und 12 liegt und das Sequestrierungsmittel der Formel (I) und das organische- oder Mineralsalz einen Komplex der Formel:

$$[N—[CHR_1—CHR_2—O—(CHR_3—CHR_4—O—)_nR_5]_3]_y\ (M^+A^-) \qquad (II)$$

bilden, in der y größer oder gleich 1 und kleiner oder gleich 3 ist ($1 \leqslant y \leqslant 3$), wobei dieser Komplex der Formel (II) in dem genannten organischen Lösungsmittel löslich ist.

2. Verfahren nach Anspruch 1, dadurch gekennzeichnet, daß man das wasserfreie organische- oder Mineralsalz und das in dem genannten organischen Lösungsmittel in Lösung vorliegende Sequestrierungsmittel der Formel (I) in einer einzigen Verfahrensstufe einsetzt.

3. Verfahren nach Anspruch 1, dadurch gekennzeichnet, daß man das organische- oder Mineralsalze in wässriger Lösung und das in dem genannten organischen Lösungsmittel in Lösung vorliegende Sequestrierungsmittel der Formel (I) in einer einzigen Verfahrensstufe einsetzt.

4. Verfahren nach Anspruch 1, dadurch gekennzeichnet, daß man in einer ersten Stufe das in einem Lösungsvermittler gelöste Mineral- oder organische Salz und das in demselben Lösungsvermittler gelöste Sequestrierungsmittel der Formel (I) einsetzt, daß man in einer zweiten Stufe den Lösungsvermittler beseitigt und daß man in einer dritten Stufe das aus der zweiten Stufe erhaltene Produkt und das genannten Lösungsmittel einsetzt.

5. Verfahren nach Anspruch 1, dadurch gekennzeichnet, daß man in einer ersten Stufe das wasserfreie organische- oder Mineralsalz und das Sequestrierungsmittel der Formel (I) in Abwesenheit irgendeines Lösungsmittels einsetzt und daß man in einer zweiten Stufe das aus der ersten Stufe resultierende Produkt und das genannte organische Lösungsmittel einsetzt.

6. Verfahren nach irgendeinem der vorstehenden Ansprüche, dadurch gekennzeichnet, daß in der Formel (I) $R_1$, $R_2$, $R_3$ und $R_4$ gleich oder unterschiedlich ein Wasserstoffatom oder einen Methylrest darstellen.

7. Verfahren nach irgendeinem der Ansprüche 1 bis 5, dadurch gekennzeichnet, daß in der Formel (I) n eine ganze Zahl größer oder gleich 0 und kleiner oder gleich 6 ist.

8. Verfahren nach irgendeinem der Ansprüche 1 bis 5, dadurch gekennzeichnet, daß in der Formel (I) $R_5$ einen Alkylrest mit 1 bis 4 Kohlenstoffatomen darstellt.

9. Verfahren nach irgendeinem der Ansprüche 1 bis 5, dadurch gekennzeichnet, daß in der Formel (I) $R_1$, $R_2$, $R_3$ und $R_4$ gleich oder unterschiedlich ein Wasserstoffatom oder einen Methylrest darstellen, n eine ganze Zahl größer oder gleich 0 und kleiner oder gleich 6 ist und $R_5$ einen Alkylrest mit 1 bis 4 Kohlenstoffatomen darstellt.

10. Verfahren nach Anspruch 9, dadurch gekennzeichnet, daß das Sequestrierungsmittel der Formel (I) tris-(Dioxa-3,6-heptyl)amin der Formel:

$$N—(CH_2—CH_2—O—CH_2—CH_2—O—CH_3)_3$$

ist.

11. Verfahren nach Anspruch 9, dadurch gekennzeichnet, daß das Sequestrierungsmittel der Formel (I) das tris-(Trioxa-3,6,9-decyl)amin der Formel:

$$N—(CH_2—CH_2—O—CH_2—CH_2—O—CH_2—CH_2—O—CH_3)_3$$

ist.

12. Verfahren nach Anspruch 9, dadurch gekennzeichnet, daß das Sequestrierungsmittel der Formel (I) das tris-(Dioxa-3,6-octyl)amin der Formel:

$$N—(CH_2—CH_2—O—CH_2—CH_2—O—C_2H_5)_3$$

ist.

13. Verfahren nach Anspruch 9, dadurch gekennzeichnet, daß das Sequestrierungsmittel der Formel (I) das tris(Trioxa-3,6,9-undecyl)amin der Formel:

$$N—(CH_2—CH_2—O—CH_2—CH_2—O—CH_2—CH_2—O—C_2H_5)_3$$

ist.

14. Verfahren nach Anspruch 9, dadurch gekennzeichnet, daß das Sequestrierungsmittel der Formel (I) das tris(Dioxa-3,6-nonyl)amin der Formel:

$$N—(CH_2—CH_2—O—CH_2—CH_2—O—C_3H_7)_3$$

ist.

15. Verfahren nach Anspruch 9, dadurch gekennzeichnet, daß das Sequestrierungsmittel der Formel (I) das tris(Trioxa-3,6,9-dodecyl)amin der Formel:

$$N—(CH_2—CH_2—O—CH_2—CH_2—O—CH_2—CH_2—O—C_3H_7)_3$$

ist.

16. Verfahren nach Anspruch 9, dadurch gekennzeichnet, daß das Sequestrierungsmittel der Formel (I) das tris(Dioxa-3,6-decyl)amin der Formel:

$$N—(CH_2—CH_2—O—CH_2—CH_2—O—C_4H_9)_3$$

ist.

17. Verfahren nach Anspruch 9, dadurch gekennzeichnet, daß das Sequestrierungsmittel der Formel (I) das tris(Trioxa-3,6,9-tridecyl)amin der Formel:

$$N—(CH_2—CH_2—O—CH_2—CH_2—O—CH_2—CH_2—O—C_4H_9)_3$$

ist.

18. Verfahren nach Anspruch 9, dadurch gekennzeichnet, daß das Sequestrierungsmittel der Formel (I) das tris(Oxa-3-butyl)amin der Formel:

$$N—(CH_2—CH_2—O—CH_3)_3$$

ist.

19. Verfahren nach Anspruch 9, dadurch gekennzeichnet, daß das Sequestrierungsmittel der Formel (I) das tris(Tetraoxa-3,6,9,12-tridecyl)amin der Formel:

$$N—(CH_2—CH_2—O—CH_2—CH_2—O—CH_2—CH_2—O—CH_2—CH_2—O—CH_3)_3$$

ist.

20. Verfahren nach Anspruch 9, dadurch gekennzeichnet, daß das Sequestrierungsmittel der Formel (I) das tris(Hexaoxa-3,6,9,12,15,18-nonadecyl)amin der Formel:

$$[N—[(CH_2—CH_2—O)_6—CH_3]_3]_y$$

ist.

21. Verfahren nach Anspruch 9, dadurch gekennzeichnet, daß das Sequestrierungsmittel der Formel (I) das tris(Dioxa-3,6-methyl-4-heptyl)amin der Formel:

$$N{-}(CH_2{-}CH_2{-}O{-}CHCH_3{-}CH_3{-}O{-}CH_3)_3$$

ist.

22. Verfahren nach Anspruch 9, dadurch gekennzeichnet, daß das Sequestrierungsmittel der Formel (I) das tris(Dioxa-3,6-dimethyl-2,4-heptyl)amin der Formel:

$$N{-}(CH_2{-}CHCH_3{-}O{-}CHCH_3{-}CH_2{-}O{-}CH_3)_3$$

ist.

23. Verfahren nach Anspruch 1, dadurch gekennzeichnet, daß das Kation $M^+$ aus der Gruppe ausgewählt ist, die das Kation $NH_4^+$, die Kationen $RNH_3^+$, wo R ein Alkylrest oder Arylrest ist und die Kationen der Metalle Li, Na, K, Rb, Cs, Mg, Ca, Sr, Ba, Sc, Y, La und die Lanthaniden, Ac und Actiniden, Ti, Zr, Hf, V, Nb, Ta, Cr, Mo, W, Mn, Tc, Re, Fe, Co, Ni, Ru, Rh, Pd, Os, Ir, Pt, Cu, Ag, Au, Zn, Cd, Hg, Al, Ga, In, Tl, Ge, Sn, Pb, Sb, Bi umfasst.

24. Verfahren nach Anspruch 1, dadurch gekennzeichnet, daß das Anion $A^-$ aus der Gruppe ausgewählt ist, die

$SCN^-$, $O{=}C{=}N^-$, $Cl^-$, $Br^-$, $I^-$, $F^-$, $CN^-$, $SH^-$, $S^=$, $OH^-$, $HSO_3^-$, $ClO_4^-$, $BrO_4^-$, $NH_2^-$,

$NO_3^-$, $NO_2^-$, $BF_4^-$, $BrO^-$, $ClO^-$, $BH_4^-$, $SO_3^{--}$, $PO_4^{3-}$, $CO_3^{--}$, $SO_4^{--}$, $ClO_3^-$, $BrO_3^-$, $H^-$, $AlH_4^-$

umfasst.

25. Verfahren nach Anspruch 1, dadurch gekennzeichnet, daß das Anion $A^-$ aus der Gruppe ausgewählt ist, die die Anionen umfasst, die von Alkoholen, Phenolen, Thiolen, Thiophenolen, Säuren, Aminen, Amiden, organischen Verbindungen mit beweglichem Wasserstoff und Silanolen stammen.

26. Verfahren nach Anspruch 1, dadurch gekennzeichnet, daß das genannte organische Lösungsmittel aus der Gruppe ausgewählt ist, die Hexan, Cyclohexan, Benzol, Toluol, o-Xylol, m-Xylol, Chlorbenzol, o-Dichlorbenzol, Trichloro-1,2,4-benzol, Chloroform, Methylenchlorid, Tetrachlorkohlenstoff, Dichloro-1,2-äthan, Trichloro-1,1,1-äthan, Trichloro-1,1,2-äthan, Trichloro-1,2,2-trifluoro-1,1,2-äthan, Perchloräthylen, Acetonitril, Dimethylformamid, N-Methylpyrrolidon, Dimethylacetamid, Hexamethylphosphorotriamid, Dimethylsulfoxid, Sulfolan, Methanol, Äthanol und Isopropanol umfasst.

27. Verfahren nach Anspruch 1, dadurch gekennzeichnet, daß man bei einer Temperatur zwischen −50°C und 250°C arbeitet.

28. Verfahren nach Anspruch 1, dadurch gekennzeichnet, daß man das Sequestrierungsmittel der Formel (I) in einer solchen Menge verwendet, daß das Molverhältnis des Sequestrierungsmittel zum Salze $A^-$ $M^+$ zwischen 0,001 und 50 liegt.

29. Anwendung des Verfahrens nach irgendeinem der vorstehenden Ansprüche zum Einbringen des Salzes $A^-$ $M^+$ in Lösung in dem genannten organischen Lösungsmittel in eine organische Synthesereaktion.

30. Anwendung des Verfahrens nach irgendeinem der Ansprüche 1 bis 28 zur Extraktion des Salzes $A^-$ $M^+$ aus einer dieses enthaltenden Lösung.

**Claims for the Contracting State: AT**

1. Process for solubilising an organic or inorganic salt in an organic solvent in which the organic or inorganic salt is initially insoluble, or for increasing the solubility of an organic or inorganic salt in an organic solvent, characterised in that the organic or inorganic salt of the formula $A^-M^+$, in which $A^-$ represents an inorganic or organic anion and $M^+$ represents a cation chosen from amongst the group comprising the cation $NH_4^+$ and its derivatives and the cations derived from the metals of groups $I_A$, $II_A$, $III_A$, $IV_A$, $V_A$, $VI_A$, $VII_A$, VIII, $I_B$, $II_B$, $III_B$, $IV_B$ and $V_B$ of the periodic classification of the elements, is brought together with at least one sequestering agent, which is soluble in the said organic solvent, of the formula:

$$N{-}[CHR_1{-}CHR_2{-}O{-}(CHR_3{-}CHR_4{-}O)_n{-}R_5]_3 \quad \text{(I)},$$

in which n is an integer which is greater than or equal to 0 and less than or equal to 10 ($0 \leq n \leq 10$), $R_1$, $R_2$, $R_3$ and $R_4$, which are identical or different, represent a hydrogen atom or an alkyl radical having from 1 to 4 carbon atoms and $R_5$ represents an alkyl or cycloalkyl radical having from 1 to 12 carbon atoms, a phenyl radical or a radical $-C_mH_{2m}-\emptyset$ or $C_mH_{2m+1}-\emptyset-$, in which m is between 1 and 12, the sequestering agent of the formula (I) and the organic or inorganic salt forming a complex of the formula:

$$[N—(CHR_1—CHR_2—O—(CHR_3—CHR_4—O—)_nR_5]_3]_y(M^+A^-) \quad (II),$$

in which y is greater than or equal to 1 and less than or equal to 3 ($1 \leqslant y \leqslant 3$), and this complex of the formula (II) being soluble in the said organic solvent.

2. Process according to Claim 1, characterised in that the anhydrous organic or inorganic salt is brought together in a single step with a solution of the sequestering agent of the formula (I) in the said organic solvent.

3. Process according to Claim 1, characterised in that an aqueous solution of the organic or inorganic salt is brought together in a single step with a solution of the sequestering agent of the formula (I) in the said organic solvent.

4. Process according to Claim 1, characterised in that, in a first step, a solution of the inorganic or organic salt in an auxiliary solvent is brought together with a solution of the sequestering agent of the formula (I) in the same auxiliary solvent, in that, in a second step, the auxiliary solvent is removed, and in that, in a third step, the product resulting from the second step is brought together with the said solvent.

5. Process according to Claim 1, characterised in that, in a first step, the anhydrous inorganic or organic salt is brought together with the sequestering agent of the formula (I) in the absence of any solvent, and in that, in a second step, the product resulting from the first step is brought together with the said organic solvent.

6. Process according to any one of the preceding claims, characterised in that, in the formula (I), $R_1$, $R_2$, $R_3$ and $R_4$, which are identical or different, represent a hydrogen atom or a methyl radical.

7. Process according to any one of Claims 1 to 5, characterised in that, in the formula (I), n is an integer which is greater than or equal to 0 and less than or equal to 6.

8. Process according to any one of Claims 1 to 5, characterised in that, in the formula (I), $R_5$ represents an alkyl radical having from 1 to 4 carbon atoms.

9. Process according to any one of Claims 1 to 5, characterised in that, in the formula (I), $R_1$, $R_2$, $R_3$ and $R_4$, which are identical or different, represent a hydrogen atom or a methyl radical, n is an integer which is greater than or equal to 0 and less than or equal to 6 and $R_5$ represents an alkyl radical having from 1 to 4 carbon atoms.

10. Process according to Claim 9, characterised in that the sequestering agent of the formula (I) is tris-(3,6)-dioxaheptyl)-amine of the formula:

$$N(CH_2—CH_2—O—CH_2—CH_2—O—CH_3)_3.$$

11. Process according to Claim 9, characterised in that the sequestering agent of the formula (I) is tris-(3,6,9-trioxadecyl)-amine of the formula:

$$N—(CH_2—CH_2—O—CH_2—CH_2—O—Ch_2—CH_2—O—CH_3)_3).$$

12. Process according to Claim 9, characterised in that the sequestering agent of the formula (I) is tris-(3,6-dioxaoctyl)-amine of the formula:

$$N—(CH_2—CH_2—O—CH_2—CH_2—O—C_2H_5)_3.$$

13. Process according to Claim 9, characterised in that the sequestering agent of the formula (I) is tris-(3,6,9-trioxaundecyl)-amine of the formula:

$$N—(CH_2—CH_2—O—CH_2—CH_2—O—CH_2—CH_2—O—C_2H_5)_3.$$

14. Process according to Claim 9, characterised in that the sequestering agent of the formula (I) is tris-(3,6-dioxanonyl)-amine of the formula:

$$N—(CH_2—CH_2—O—CH_2—CH_2—O—C_3H_7)_3.$$

15. Process according to Claim 9, characterised in that the sequestering agent of the formula (I) is tris-(3,6,9-trioxadodecyl)-amine of the formula:

$$N—(CH_2—CH_2—O—CH_2—CH_2—O—CH_2—CH_2—O—C_3H_7)_3.$$

16. Process according to Claim 9, characterised in that the sequestering agent of the formula (I) is tris-(3,6-dioxadecyl)-amine of the formula:

$$N—(CH_2—CH_2—O—CH_2—CH_2—O—C_4H_9)_3).$$

17. Process according to Claim 9, characterised in that the sequestering agent of the formula (I) is tris-(3,6,9-trioxatridecyl)-amine of the formula:

$$N—(CH_2—CH_2—O—CH_2—CH_2—O—CH_2—CH_2—O—C_4H_9)_3.$$

18. Process according to Claim 9, characterised in that the sequestering agent of the formula (I) is tris-(3-oxabutyl)-amine of the formula:

$$N—(CH_2—CH_2—O—CH_3)_3.$$

19. Process according to Claim 9, characterised in that the sequestering agent of the formula (I) is tris-(3,6,9,12-tetraoxatridecyl)-amine of the formula:

$$N—(CH_2—CH_2—O—CH_2—CH_2—O—CH_2—CH_2—O—CH_2—CH_2—O—CH_3)_3.$$

20. Process according to Claim 9, characterised in that the sequestering agent of the formula (I) is tris-(3,6,9,12,15,18-hexaoxanonadecyl)-amine of the formula:

$$[N—[(CH_2—CH_2—O)_6—CH_3]_3]_y.$$

21. Process according to Claim 9, characterised in that the sequestering agent of the formula (I) is tris-(3,6-dioxa-4-methylheptyl)-amine of the formula:

$$N—(CH_2—CH_2—O—CHCH_3—CH_2—O—CH_3)_3.$$

22. Process according to Claim 9, characterised in that the sequestering agent of the formula (I) is tris-(3,6-dioxa-2,4-dimethylheptyl)-amine of the formula:

$$N—(CH_2—CHCH_3—O—CHCH_3—CH_2—O—CH_3)_3.$$

23. Process according to Claim 1, characterised in that the cation $M^+$ is chosen from amongst the group comprising the cation $NH_4^+$, the cations $RNH_3^+$, in which R is an alkyl or aryl radical, and the cations derived from the metals Li, Na, K, Rb, Cs, Mg, Ca, Sr, Ba, Sc, Y, La and lanthanides, Ac and actinides, Ti, Zr, Hf, V, Nb, Ta, Cr, Mo, W, Mn, Tc, Re, Fe, Co, Ni, Ru, Rh, Pd, Os, Ir, Pt, Cu, Ag, Au, Zn, Cd, Hg, Al, Ga, In, Tl, Ge, Sn, Pb, Sb and Bi.

24. Process according to Claim 1, characterised in that the anion $A^-$ is chosen from amongst the group comprising

$$SCN^-, O{=}C{=}N^-, Cl^-, Br^-, I^-, F^-, CN^-, SH^-, S^{--}, OH^-, HSO_3^-, ClO_4^-, BrO_4^-,$$

$$NH_2^-, NO_3^-, NO_2^-, BF_4^-, BrO^-, ClO^-, BH_4^-, SO_3^{--}, PO_4^{3-}, CO_3^{--}, SO_4^{--}, ClO_3^-, BrO_3^-, H^-, \text{and } AlH_4^-.$$

25. Process according to Claim 1, characterised in that the anion $A^-$ is chosen from amongst the group comprising the anions derived from alcohols, phenols, thiols, thiophenols, acids, amines, amides, organic compounds with a mobile hydrogen, and silanols.

26. Process according to Claim 1, characterised in that the said organic solvent is chosen from amongst the group comprising hexane, cyclohexane, benzene, toluene, o-xylene, m-xylene, p-xylene, chlorobenzene, o-dichlorobenzene, 1,2,4-trichlorobenzene, chloroform, methylene chloride, carbon tetrachloride, 1,2-dichloroethane, 1,1,1-trichloroethane, 1,1,2-trichloroethane, 1,2,2-trichloro-1,1,2-trifluoroethane, perchloroethylene, acetonitrile, dimethylformamide, N-methylpyrrolidone, dimethylacetamide, hexamethylphosphorotriamide, dimethyl sulphoxide, sulpholane, methanol, ethanol and isopropanol.

27. Process according to Claim 1, characterised in that the reaction is carried out at a temperature between −50°C and 250°C.

28. Process according to Claim 1, characterised in that the sequestering agent of the formula (I) is used in amounts such that the molar ratio of the sequestering agent to the salt $A^-M^+$ is between 0.001 and 50.

29. Application of the process according to any one of the preceding claims to the use of a solution of the salt $A^-M^+$ in the said organic solvent, in an organic synthesis reaction.

30. Application of the process according to any one of Claims 1 to 28 to the extraction of the salt $A^-M^+$ from a solution in which it is present.